# EUROPEAN PATENT APPLICATION

(11) **EP 4 194 538 A1**
(43) Date of publication of application: **14.06.2023**
(21) Application number: 21854067.2
(22) Date of filing: 05.08.2021
(51) Int. Cl.: C12M 1/00, C12M 1/28, C12N 15/10, C12Q 1/6806, G01N 1/00, G01N 37/00

(54) **NUCLEIC ACID EXTRACTION CONTAINER AND NUCLEIC ACID EXTRACTION METHOD**

(30) Priority: 07.08.2020 JP 2020134388
(71) Applicant: Go!Foton, Inc., Tsukuba City, Ibaraki 300-2635 (JP)
(72) Inventor: FUKUZAWA Takashi, Machida-shi Tokyo 194-0212 (JP)
(74) Representative: Algemeen Octrooi- en Merkenbureau B.V.
(86) International application number: PCT/JP2021/029228
(87) International publication number: WO 2022/030605

(57) **Abstract**

A nucleic acid extraction container 160 that is provided with a filter part 166, wherein the filter part 166 comprises a hydrophilic filter 168 for collecting biological materials including a nucleic acid from a sample, a biological material collection area 166a for collecting the biological materials on the filter 168, and a sample permeation area 166b for allowing the passage of the sample having permeated through the filter. A sample injection port 174 is communicated with the biological material collection area 166a. An air communication port 178 is communicated with the biological material collection area 166a. A sample discharge port 176 is communicated with the sample permeation area 166b. The air communication port 178 is configured so as to be outwardly openable and closable.

## Description

### [TECHNICAL FIELD]

The present invention relates to a nucleic acid extraction container and a nucleic acid extraction method.

### [BACKGROUND ART]

Genetic testing is widely used for examinations in a wide variety of medical fields, identification of farm products and pathogenic microorganisms, safety assessment for food products, and even for examinations for pathogenic viruses and a variety of infectious diseases. In order to detect with high sensitivity a minute amount of a nucleic acid constituting genes, methods of analyzing the resultant obtained by amplifying a portion of a nucleic acid are known. Among them, polymerase chain reaction (PCR) is a remarkable technique for selectively amplifying a certain portion of a nucleic acid in a very small amount collected from a living organism or the like. In order to perform this PCR, it is necessary to extract a nucleic acid from a biological sample.

As an example of such genetic testing, in order to determine whether a specific organism can live in environments such as water, soil, and air, a technology is known that analyzes the presence or absence of DNA derived from a specific organism (referred to as environmental DNA) by PCR (see, for example, Non-Patent Literatures 1 to 3). For analysis, the eDNA Society has prepared "Environmental DNA Sampling and Experiment Manual" and recommends that this method (hereinafter, referred to as the society's standard method) be used as a standard method for qualitative and quantitative measurements (see Non-Patent Literature 4). According to the society's standard method, DNA is extracted after pumping river or sea water with a beaker or the like and filtering the water.
[Non-Patent Literature 1] Hideyuki Doi et.al, Freshwater Biology (2017) 62, 30-39
[Non-Patent Literature 2] PLOS ONE | DOI:10.1371/journal.pone.0149786 March 2, 2016
[Non-Patent Literature 3] PLOS ONE | DOI:10.1371/journal.pone.0142008 November 4, 2015
[Non-Patent Literature 4] "Environmental DNA Sampling and Experiment Manual (ver2.1)", The eDNA Society, Internet <http://ednasociety.org/eDNA_manual_ver2_1_3.pdf>

### [SUMMARY OF INVENTION]

### [TECHNICAL PROBLEM]

The society's standard method allows for measurement results to be obtained relatively in a reliable manner and is considered to be a highly efficient method (the ratio of DNA extraction from the environment to a state where it can be subjected to amplification such as PCR). However, this method comes with the following problems: the extraction of DNA from a sample requires many steps and is thus time-consuming, skills are required for the extraction, measurement results wary widely among operators, and there are many parts, equipment, and reagents required for the extraction that are expensive. These problems are not limited to environmental DNA and are also seen in the extraction of a nucleic acid for identification of microorganisms and viruses.

In this background, one of the purposes of the present invention is to provide a technology that allows extraction of a nucleic acid from a sample to be performed easily in a short period of time and at a low cost.

### [SOLUTION TO PROBLEM]

One embodiment of the present invention relates to a nucleic acid extraction container. This nucleic acid extraction container includes:
a filter part that includes a hydrophilic filter for collecting biological materials containing a nucleic acid from a sample, a biological material collection area for collecting the biological materials on the filter, and a sample permeation area for allowing the passage of the sample having permeated through the filter;
a sample injection port that communicates with the biological material collection area;
an air communication port that communicates with the biological material collection area; and
a sample discharge port that communications with the sample permeation area, wherein
the air communication port is configured so as to be outwardly openable and closable.

Another embodiment of the present invention relates to a nucleic acid extraction method. This nucleic acid extraction method includes:
passing a sample through a filter part provided with a hydrophilic filter and collecting biological materials containing a nucleic acid on the filter;
putting a nucleic acid extraction solution in the filter part and extracting the nucleic acid on the filter; and
collecting a solution containing the nucleic acid extracted on the filter.

Yet another embodiment of the present invention also relates to a nucleic acid extraction method. This nucleic acid extraction method includes:
passing a sample through a filter part provided with a hydrophilic filter and collecting biological materials containing a nucleic acid on the filter;
putting a nucleic acid extraction solution in the filter part and extracting the nucleic acid on the filter; and
moving a solution containing the nucleic acid extracted on the filter into a channel communicating with the filter part; and
dispensing the solution containing the nucleic acid by injecting air in the channel.

### [ADVANTAGEOUS EFFECTS OF INVENTION]

According to the present invention, extraction of a nucleic acid from a sample can be performed easily in a short period of time and at a low cost.

### [BRIEF DESCRIPTION OF DRAWINGS]

FIGS. 1A and 1B are diagrams for explaining a nucleic acid extraction container according to the first embodiment of the present invention;
FIG. 2 is a cross-sectional view of the nucleic acid extraction container shown in FIG. 1A that is sectioned along line A-A;
FIG. 3 is a cross-sectional view of the nucleic acid extraction container shown in FIG. 1A that is sectioned along line B-B;
FIG. 4 is a plan view of a substrate provided in the nucleic acid extraction container shown in FIG. 1A;
FIG. 5 is a diagram for explaining the first exemplary variation of a nucleic acid extraction container;
FIG. 6 is a diagram for explaining the second exemplary variation of a nucleic acid extraction container;
FIG. 7 is a diagram for explaining the flow of a sample passing through a filter part;
FIG. 8 is a diagram for explaining nucleic acid extraction on a filter by a nucleic acid extraction solution; and
FIGS. 9A and 9B are diagrams for explaining a nucleic acid extraction container according to the second embodiment of the present invention;
FIG. 10 is a cross-sectional view of the nucleic acid extraction container shown in FIG. 9A that is sectioned along line A-A;
FIG. 11 is a cross-sectional view of the nucleic acid extraction container shown in FIG. 9A that is sectioned along line B-B;
FIG. 12 is a cross-sectional view of the nucleic acid extraction container shown in FIG. 9A that is sectioned along line C-C;
FIG. 13 is a cross-sectional view of the nucleic acid extraction container shown in FIG. 9A that is sectioned along line D-D;
FIG. 14 is a plan view of a substrate provided in the nucleic acid extraction container shown in FIG. 9A;
FIGS. 15A and 15B are diagrams for explaining a nucleic acid extraction container according to the third embodiment of the present invention;
FIG. 16 is a cross-sectional view of the nucleic acid extraction container shown in FIG. 15A that is sectioned along line A-A;
FIG. 17 is a cross-sectional view of the nucleic acid extraction container shown in FIG. 15A that is sectioned along line E-E;
FIG. 18 is a plan view of a substrate provided in the nucleic acid extraction container shown in FIG. 15A;
FIG. 19 is a diagram schematically showing a state where a solution containing a nucleic acid is in a region C of a third channel;
FIGS. 20A and 20B are diagrams for explaining a nucleic acid extraction container according to the fourth embodiment of the present invention;
FIG. 21 is a cross-sectional view of the nucleic acid extraction container shown in FIG. 20A that is sectioned along line A-A;
FIG. 22 is a cross-sectional view of the nucleic acid extraction container shown in FIG. 20A that is sectioned along line B-B;
FIG. 23 is a cross-sectional view of the nucleic acid extraction container shown in FIG. 20A that is sectioned along line C-C;
FIG. 24 is a cross-sectional view of the nucleic acid extraction container shown in FIG. 20A that is sectioned along line D-D;
FIG. 25 is a plan view of a substrate provided in the nucleic acid extraction container shown in FIG. 20A;
FIGS. 26A and 26B are diagrams for explaining a nucleic acid extraction container according to the fifth embodiment of the present invention;
FIG. 27 is a cross-sectional view of the nucleic acid extraction container shown in FIG. 26A that is sectioned along line A-A;
FIG. 28 is a cross-sectional view of the nucleic acid extraction container shown in FIG. 26A that is sectioned along line B-B;
FIG. 29 is a cross-sectional view of the nucleic acid extraction container shown in FIG. 26A that is sectioned along line C-C;
FIG. 30 is a cross-sectional view of the nucleic acid extraction container shown in FIG. 26A that is sectioned along line D-D;
FIG. 31 is a cross-sectional view of the nucleic acid extraction container shown in FIG. 26A that is sectioned along line E-E;
FIG. 32 is a cross-sectional view of the nucleic acid extraction container shown in FIG. 26A that is sectioned along line F-F;
FIG. 33 is a plan view of a substrate provided in the nucleic acid extraction container shown in FIG. 26A;
FIGS. 34A and 34B are diagrams for explaining a pressing plate provided in a nucleic acid extraction container according to the fifth embodiment of the present invention;
FIG. 35 is a cross-sectional view of the pressing plate shown in FIG. 34A that is sectioned along line J-J;
FIG. 36 is a cross-sectional view of the pressing plate shown in FIG. 34A that is sectioned along line H-H;
FIG. 37 is a cross-sectional view of the pressing plate shown in FIG. 34A that is sectioned along line G-G or line I-I; and
FIG. 38 is a graph showing the relationship between the amount of an extraction solution and DNA concentration when the volume of a biological material collection area is 20 µL.

### [DESCRIPTION OF EMBODIMENTS]

Described below is an explanation of the present invention based on the attached figures and preferred embodiments. The embodiments do not limit the invention and are shown for illustrative purposes, and not all the features described in the embodiments and combinations thereof are necessarily essential to the invention.

### [First Embodiment]

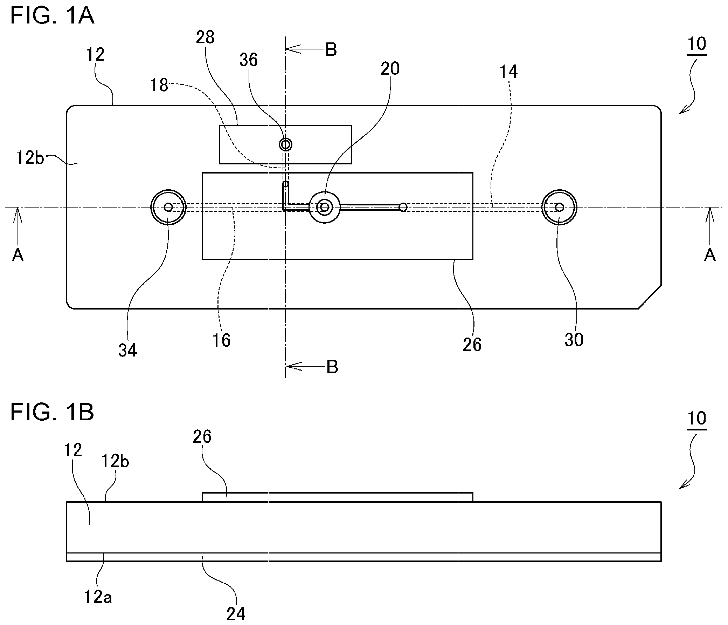

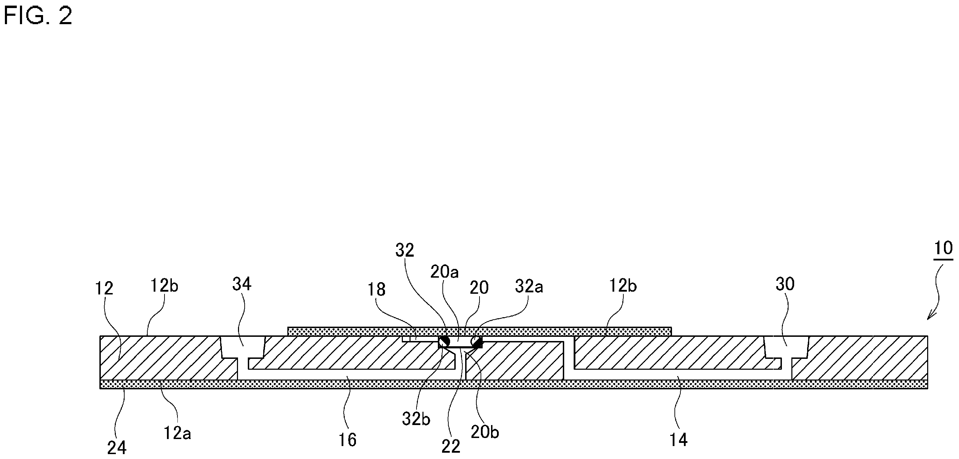

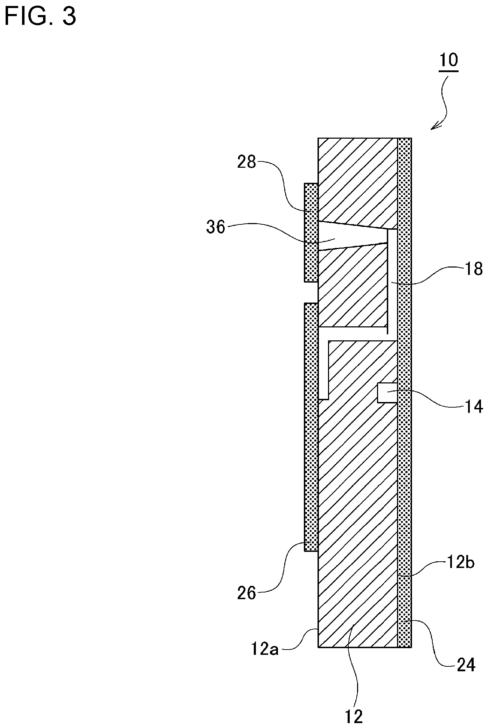

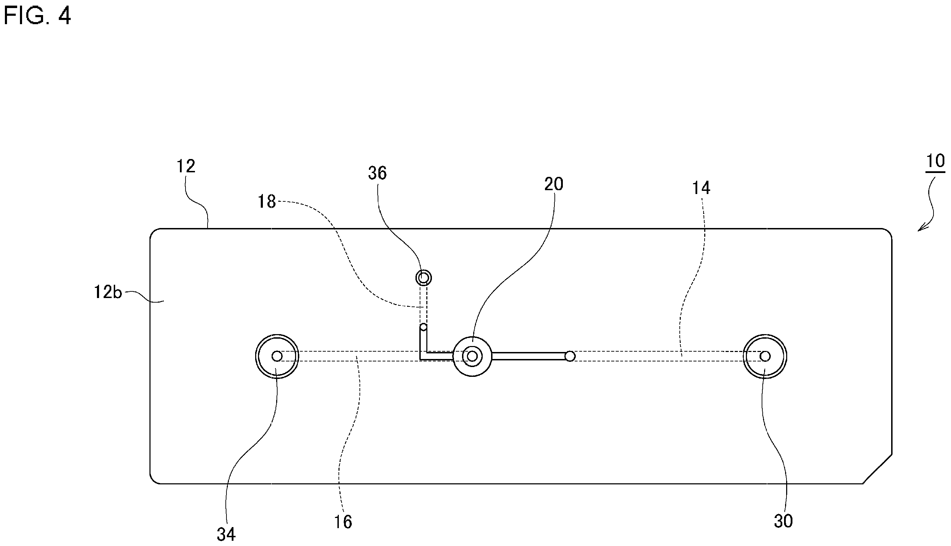

FIGS. 1A and 1B are diagrams for explaining a nucleic acid extraction container 10 according to the first embodiment of the present invention. FIG. 1A is a plan view of the nucleic acid extraction container 10, and FIG. 1B is a front view of the nucleic acid extraction container 10. FIG. 2 is a cross-sectional view of the nucleic acid extraction container shown in FIG. 1A that is sectioned along line A-A. FIG. 3 is a cross-sectional view of the nucleic acid extraction container shown in FIG. 1A that is sectioned along line B-B. FIG. 4 is a plan view of a substrate provided in the nucleic acid extraction container shown in FIG. 1A.

The nucleic acid extraction container 10 includes: a resinous substrate 12 having a groove-like first channel 14, a second channel 16, a third channel 18, and a filter part 20 provided with a hydrophilic filter 22 that are formed on a lower surface 12a and an upper surface 12b thereof; a first sealing film 24, which is attached on the lower surface 12a of the substrate 12, for sealing a portion of the first channel 14 and the second channel 16; and two sealing films (a second sealing film 26 and a third sealing film 28) attached on the upper surface 12b of the substrate 12.

The substrate 12 is preferably formed of a material that is stable under temperature changes and is resistant to a sample solution that is used. Further, the substrate 12 is preferably formed of a material that has good moldability, a good transparency and barrier property, and a low self-fluorescent property. As such a material, an inorganic material such as glass or the like, resin such as polypropylene, acrylic, polyester, silicone, or the like, and particularly a cycloolefin polymer resin (COP) are preferred. An example of the dimensions of the substrate 12 is 72 mm on the long side, 26 mm on the short side, and 4 mm in thickness. As a size of the substrate 12 that is easy to work with, a long side of 50 to 200 mm is particularly preferred. From the viewpoint of moldability, a thickness of 1 to 4 mm is preferred. For the short side, 10 to 50 mm is preferred from the viewpoint of molding cost. Such a substrate can be produced by injection molding, cast molding, or cutting and processing with an NC processing machine or the like.

The first sealing film 24 and the second sealing film 26 may be sticky on one of the main surfaces thereof or may have a functional layer that exhibits stickiness or adhesiveness through pressing, energy irradiation with ultraviolet rays or the like, heating, etc., formed on one of the main surfaces. Thus, the first sealing film 24 and the second sealing film 26 have a function of being able to become integral with the lower surface 12a and the upper surface 12b of the substrate 12 easily while being in close contact with the surfaces. The first sealing film 24 and the second sealing film 26 are desirably formed of a material, including an adhesive, that has a low autofluorescence property. In this respect, a transparent film made of resin such as a cycloolefin polymer, polyester, polypropylene, polyethylene or acrylic is suitable but is not limited thereto. Further, the first sealing film 24 and the second sealing film 26 may be formed of a plate-like glass or resin. Since rigidity can be expected in this case, the first sealing film 24 and the second sealing film 26 are useful for preventing warpage and deformation of the nucleic acid extraction container 10. The portions of the first sealing film 24 and the second sealing film 26 that come in contact with a channel preferably have low protein adsorption.

On the lower surface 12a and the upper surface 12b of the substrate 12, a groove-like first channel 14, a second channel 16, and a third channel 18 are formed. In the nucleic acid extraction container 10 according to the present embodiment, most parts of the first channel 14, the second channel 16, and the third channel 18 are formed in the shape of a groove exposed on the lower surface 12a and the upper surface 12b of the substrate 12. This is for allowing for inexpensive and easy molding by injection molding using a metal mold or the like. In order to seal this groove so as to make use of the groove as a channel, the first sealing film 24 is attached on the lower surface 12a of the substrate 12, and the second sealing film 26 is attached on the upper surface 12b. An example of the dimensions of the first channel 14 and the second channel 16 is 1.0 mm in width and 1.0 mm in depth. An example of the dimensions of the third channel 18 is 0.6 mm in width and 0.6 mm in depth. From the viewpoint of reducing the overall volume of the third channel, the average cross-sectional area of the third channel 18 is preferably smaller than the average cross-sectional area of the first channel 14. This allows pressure to be applied to the first channel 14 during filtering, reducing the amount of a sample entering the third channel 18. Thus, an extraction solution can be prevented from remaining in the third channel 18 when the extraction solution is collected.

The first channel 14 communicates a sample injection port 30 with the biological material collection area 20a of the filter part 20. The sample injection port 30 is formed so as to be exposed on the upper surface 12b of the substrate 12. The sample injection port 30 may be formed so as to fit well with a syringe. For example, a cylindrical tube can be extended from the substrate 12. In order to allow for stable fixing of a syringe containing the sample, the sample injection port 30 may be formed such that the sample injection port 30 can be connected to the syringe in a luer-lock manner.

The sample is introduced from the sample injection port 30 into the first channel 14. A pre-filter for removing foreign matter with a pore diameter larger than that of the filter 22 may be provided in the first channel 14.

A circular filter part 20 is formed so as to be exposed on the upper surface 12b of the substrate 12. The filter part 20 is sealed by the second sealing film 26. A circular hydrophilic filter 22 is installed in the filter part 20. The filter part is preferably formed such that the presence or absence of a sample on the filter 22 is visible from the outside during nucleic acid extraction. For example, as the second sealing film 26, a transparent film can be employed. As shown in FIG. 2, the filter part 20 includes a biological material collection area 20a for collecting biological materials containing a nucleic acid on the filter 22 and a sample permeation area 20b for allowing the passage of a sample having permeated through the filter 22. If the filter 22 is in close proximity to a hydrophilic substance in the sample permeation area 20b, water tends to remain between the filter 22 and this substance when the sample is discharged from the channel by injecting air after the biological materials are collected by the filter 22, and the remaining water makes it easier for the nucleic acid extraction solution to pass through the filter 22 at the time of the nucleic acid extraction. Therefore, the filter 22 is desirably not in close proximity to a hydrophilic substance in the sample permeation area 20b.

The filter 22 is hydrophilic. Materials for such filters include, for example, polyvinylidene fluoride (PVDF), glass fiber, polyethersulfone (PES), polyester, and cellulose. Among them, PVDF and PES, which hardly adsorb proteins, are particularly preferred. The pore diameter of the filter 22 can be selected appropriately according to the biological material to be collected. For example, the pore diameter of the filter 22 is 1 um or less.

Note that the nucleic acid include DNA, RNA, and their derivatives. Further, the biological materials include microorganisms such as bacteria and viruses in addition to substances that constitute living bodies of organisms.

In the biological material collection area 20a, the filter 22 is fixed by an O-ring 32. An example of the dimensions of the filter is 4 mm in diameter, and an example of the dimensions of the O-ring 32 is 4 mm in outer diameter and 2 mm in inner diameter. As shown in FIG. 2, notches 32a and 32b are formed in the O-ring 32 in order to send the sample from the first channel 14 onto the filter 22 and send the nucleic acid extraction solution from the third channel 18 onto the filter 22. The notch 32a on the first channel side is formed on the upper side of the O-ring 32. Alternatively, the notch 32a may be formed on the lower side of the O-ring 32. The notch 32b on the third channel side is formed on the lower side of the O-ring 32. This is to allow all of the nucleic acid extraction solution on the filter 22 to be collected.

The second channel 16 communicates a sample discharge port 34 with the sample permeation area 20b such that the liquid that has passed through the filter 22 passes through the sample permeation area 20b and then passes through the second channel so as to be discharged from the sample discharge port 34. As shown in FIG. 2, in the present embodiment, the sample discharge port 34 is formed so as to be exposed on the upper surface 12b of the substrate 12. Alternatively, the sample discharge port 34 may be formed so as to be exposed on the lower surface 12a.

The third channel 18 communicates an air communication port 36 with the biological material collection area 20a of the filter part 20. The air communication port 36 is formed so as to be exposed on the upper surface 12b of the substrate 12. As shown in FIG. 1A, a third sealing film 28 is attached to the air communication port 36. As the third sealing film 28, a sealing film having a size that allows for the sealing of the air communication port 36 is used. When a sample is allowed to permeate through the filter 22 from the sample injection port 30 via the first channel, the air communication port 36 is closed by the third sealing film 28 such that the sample does not enter the third channel. When the biological material collected on the filter 22 is extracted by a nucleic acid extraction solution, the third sealing film 28 is peeled off so as to open the air communication port 36. Configuring the air communication port so as to be outwardly openable and closable as described allows for the extraction of a nucleic acid on the filter 22. In the present embodiment, the opening and closing of the air communication port 36 is performed by the third sealing film. Alternatively, the opening and closing can also be performed by other methods such as inserting a resin or metal plug.

The nucleic acid extraction solution can be introduced using, for example, a pipettor to the biological material collection area 20a of the filter part 20 from either of the sample injection port 30 and the air communication port 36. The amount of the nucleic acid extraction solution only needs to be an amount that allows the solution to come into contact with the entire surface of the filter 22 or an amount that allows the solution to come into contact with the entire surface by moving the extraction solution in contact with a portion of the filter. The extraction solution brought into contact with the surface of the filter 22 can be collected from either of the air communication port 36 and the sample injection port 30 using, for example, a pipettor. The air communication port 36 may be formed so as to fit with the tip of the pipettor.

As shown in FIG. 1A, the first channel 14 and the third channel 18 preferably extend on opposite sides of each other across the biological material collection area 20a, and the first channel 14 and the third channel 18 in communication with the filter part 20 are more preferably located in a straight line. Thereby, the sample can be prevented from remaining in the first channel 14 and the filter part 20 after being passed through the filter 22.

In the nucleic acid extraction container 10, all channels are formed in a straight line. However, the form of the channels is not limited to this. For example, the channels may be formed in a so-called serpiginous manner where a turn is continuously made by combining curved portions and straight portions or may be widened in the middle. A plurality of filter parts 20 may be arranged in series. This can increase the area occupied by the filter parts 20 on the substrate 12 and allows for the collection of more biological materials.

### (First Exemplary Variation)

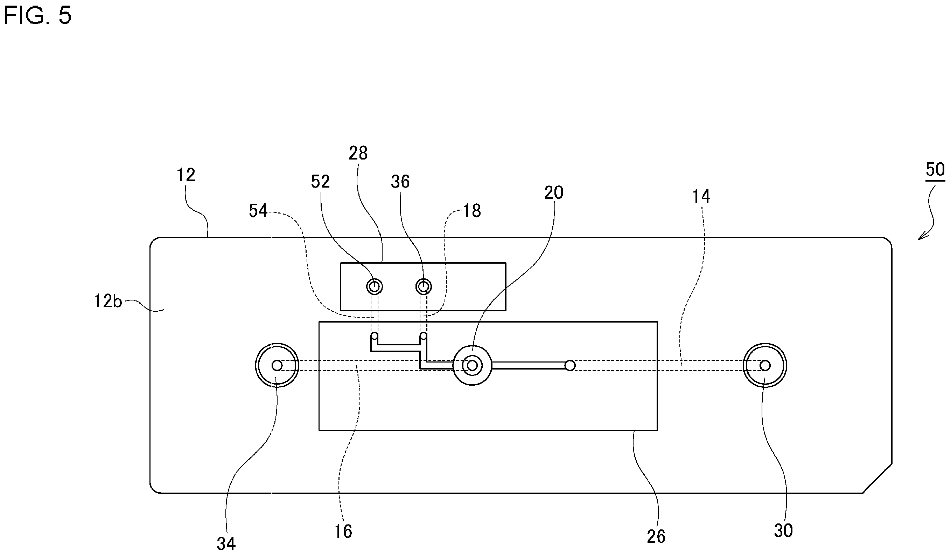

Next, an exemplary variation of the nucleic acid extraction container according to the first embodiment will be explained. FIG. 5 is a diagram for explaining the first exemplary variation of a nucleic acid extraction container. The nucleic acid extraction container 50 shown in FIG. 5 is further provided with a nucleic acid extraction solution injection port 52 on the upper surface 12b of the substrate 12, and is different from the nucleic acid extraction container 10 shown in FIG. 1A in that a first branch channel 54 communicating with the nucleic acid extraction solution injection port 52 is connected to the third channel 18. The first branch channel 54 may be connected to the first channel 14.

### (Second Exemplary Variation)

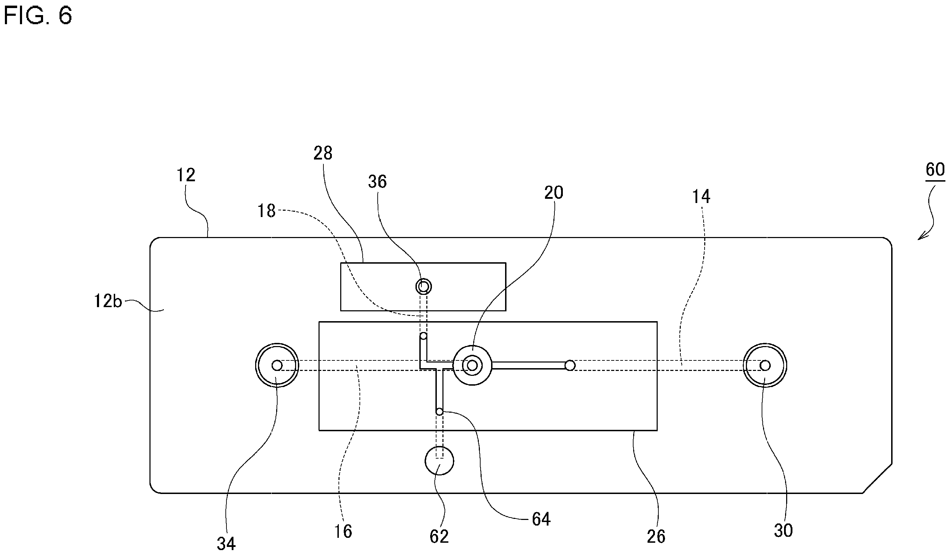

FIG. 6 is a diagram for explaining the second exemplary variation of a nucleic acid extraction container. The nucleic acid extraction container 60 shown in FIG. 6 is further provided with a nucleic acid extraction solution outlet 62 on the upper surface 12b of the substrate 12, and is different from the nucleic acid extraction container 10 shown in FIG. 1A in that a second branch channel 64 communicating with the nucleic acid extraction solution outlet 62 is connected to the third channel 18. The second branch channel 64 may be connected to the first channel 14.

### (Third Exemplary variation)

In the embodiment, the above-mentioned first and second exemplary variations may be combined so as to provide the substrate 12 with a nucleic acid extraction solution injection port, a nucleic acid extraction solution outlet, a first branching channel, and a second branch channel.

### (Fourth Exemplary variation)

In the embodiment, the nucleic acid extraction solution may be enclosed in advance in either the third channel or the first channel, and air may be enclosed between a region where the nucleic acid extraction solution is present and the filter part. This can further simplify the process of nucleic acid extraction.

### (Fifth Exemplary variation)

In the embodiment, in the sample permeation area of the filter part, a filter with a large pore diameter may be used if an object to be collected is large. In this case, although the nucleic acid extraction solution passes through the filter, applying weak pressure from the sample discharge port side can prevent the nucleic acid extraction solution from passing through the filter. The pressure applied at this time only needs to be any pressure that does not allow air to pass through a wet hydrophilic filter. For example, a micro blower pump (MZB1001T02) manufactured by Murata Manufacturing Co., Ltd., or the like can be used to apply a pressure of about 1 kP from the sample discharge port. The substrate may be formed with the channels and the filter part in such a manner that a sample passes through the filter from the lower surface of the substrate to the upper surface and the nucleic acid extraction solution passes through the lower side of the filter.

### (Sixth Exemplary Variation)

In the embodiment, one channel including the first channel, the filter part, the second channel, and the channel is formed on a single substrate. Considering the cost per channel, multiple channels are preferably formed on a single substrate.

An explanation will be given next regarding a method of using the nucleic acid extraction container 10 formed as described above. First, a sample is introduced from the sample injection port 30 into the first channel 14 and is allowed to permeate through the filter part 20. The method for introducing a sample is not limited to this. Alternatively, for example, an appropriate amount of the sample may be directly introduced through the injection port using a pipette, a dropper, a syringe, or the like. Furthermore, the sample may be moved to the filter part by discharging and introducing the sample by a pipette, a dropper, a syringe, or the like and then further pushing the sample through pressurization or sucking the sample from the sample discharge port 34.

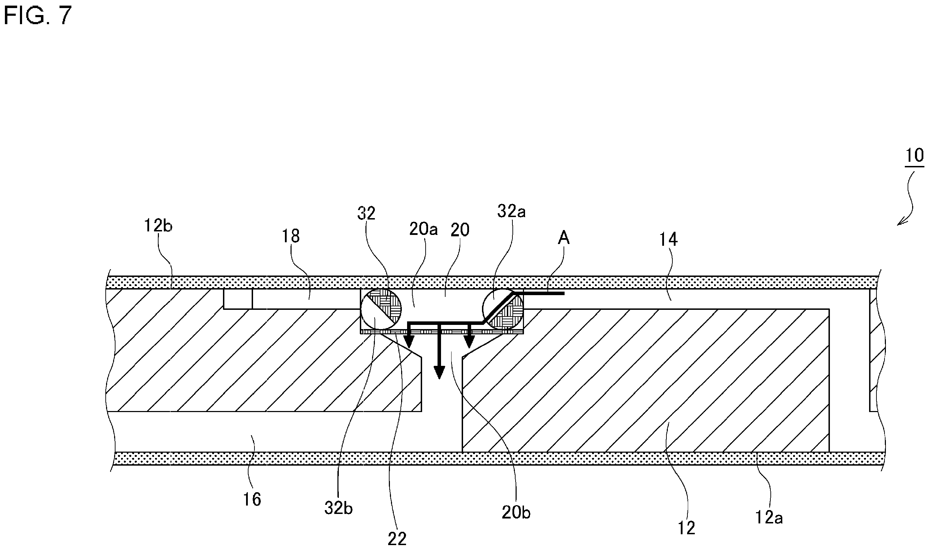

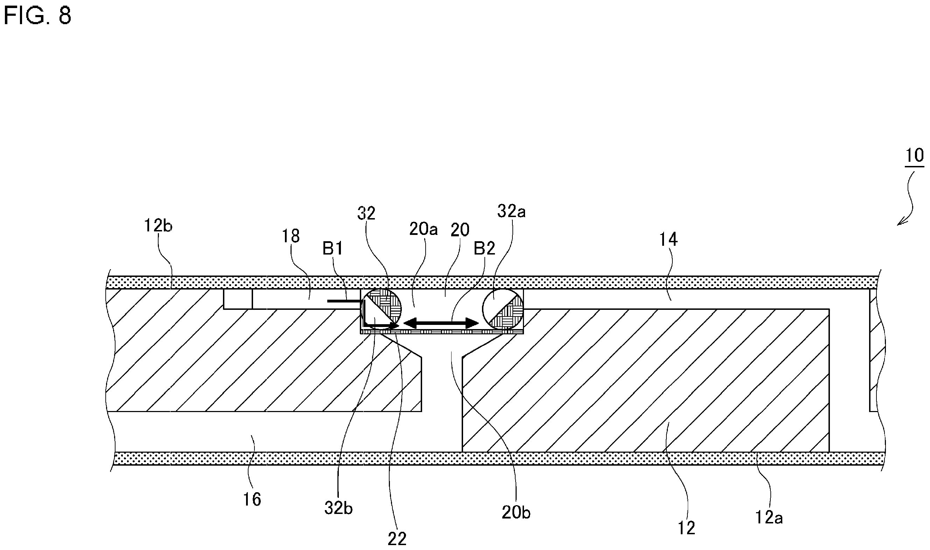

FIG. 7 is a diagram for explaining the flow of a sample passing through the filter part. As indicated by an arrow A in FIG. 8, the sample moves from the first channel 14 to the filter part 20 and passes through the filter 22. At this time, biological materials containing a nucleic acid are collected on the filter 22 in the biological material collection area 20a of the filter part 20. The sample having permeated through the filter 22 moves to the second channel 16. Next, air is injected from the sample injection port 30, and the sample is discharged from the first channel 14, the filter part 20, and the second channel 16. During these processes, the air communication port 36 remains sealed by the third sealing film 28.

Next, the third sealing film 28 is peeled off, and the nucleic acid extraction solution is injected into the third channel from the air communication port 36. FIG. 8 is a diagram for explaining nucleic acid extraction on the filter by the nucleic acid extraction solution. As indicated by an arrow B1 in FIG. 8, the nucleic acid extraction solution moves from the third channel to the filter part 20 and comes into contact with the surface of the filter 22. The nucleic acid extraction solution may be injected from the sample injection port 30 into the first channel 14 and moved to the filter part 20.

Since the pore diameter of the filter 22 is, for example, 1 um or less as described above, the nucleic acid extraction solution does not pass through the filter 22 and stays on the filter 22. The amount of the nucleic acid extraction solution may be an amount that allows the nucleic acid extraction solution to come into contact with the entire surface of the filter 22 or an amount that does not allow the nucleic acid extraction solution to come into contact with the entire surface. If the amount of the nucleic acid extraction solution is sufficient for the nucleic acid extraction solution to come into contact with the entire surface of the filter 22, leaving the nucleic acid extraction solution to stand on the filter 22 for, for example, one minute allows a nucleic acid to be extracted from the biological materials on the filter 22. If the amount of the nucleic acid extraction solution is not sufficient for the nucleic acid extraction solution to come into contact with the entire surface of the filter 22, for example, a pipettor is inserted into the air communication port 36 or the sample injection port 30, and the nucleic acid extraction solution is moved back and forth on the filter 22 by pushing or sucking air through the pipettor as shown by an arrow B2 in FIG. 8. This reciprocating motion of the nucleic acid extraction solution allows for the extraction of a nucleic acid from biological materials collected on the filter 22. The reciprocating motion of the nucleic acid extraction solution performed by the pipettor can also be performed using a microblower pump or the like. This reciprocating motion can be performed, for example, by using the method described in Japanese Patent Application Publication No. 2019-180418.

For example, if the sample contains many broken cells and mitochondria and a nucleic acid are released from the cells, the nucleic acid collected on the filter 22 are extracted when being brought into contact with the nucleic acid extraction solution at room temperature. On the other hand, if the sample is bacteria or the like, a nucleic acid may not be extracted at room temperature. In such a case, the nucleic acid can be extracted by applying a temperature of about 95 degrees Celsius. For example, by placing the substrate 12 on a high-temperature metal plate and raising the temperature of the biological material collection area 20a to around 95 degrees Celsius, a nucleic acid can be extracted from a sample such as bacteria.

The solution containing the extracted nucleic acid on the filter 22 is collected from the filter part 20 by inserting a pipettor into the air communication port 36 or the sample injection port 30 so as to suck out the solution. This completes the extraction of the nucleic acid in the sample.

### [Second Embodiment]

A nucleic acid extraction container according to the second embodiment of the present invention also includes a substrate, a sealing film attached to the substrate, and a filter. The same components as those of the nucleic acid extraction container 10 according to the first embodiment are denoted by the same reference numerals, and redundant description will be omitted as appropriate.

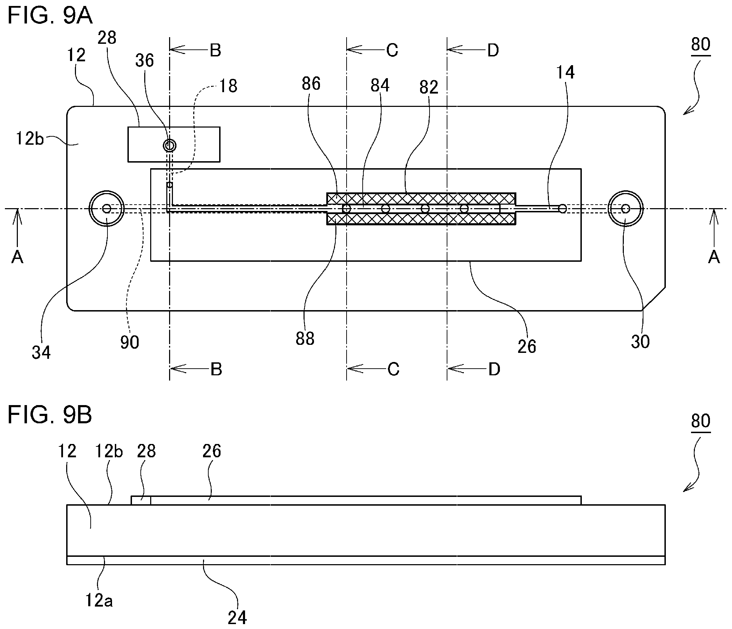

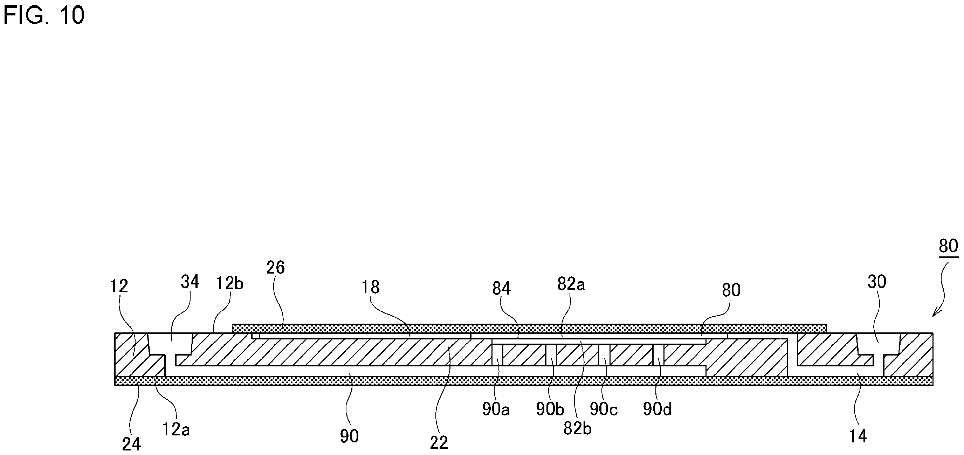

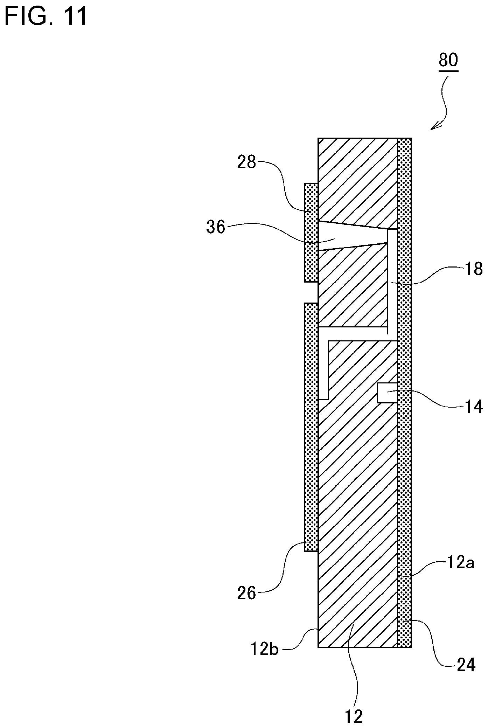

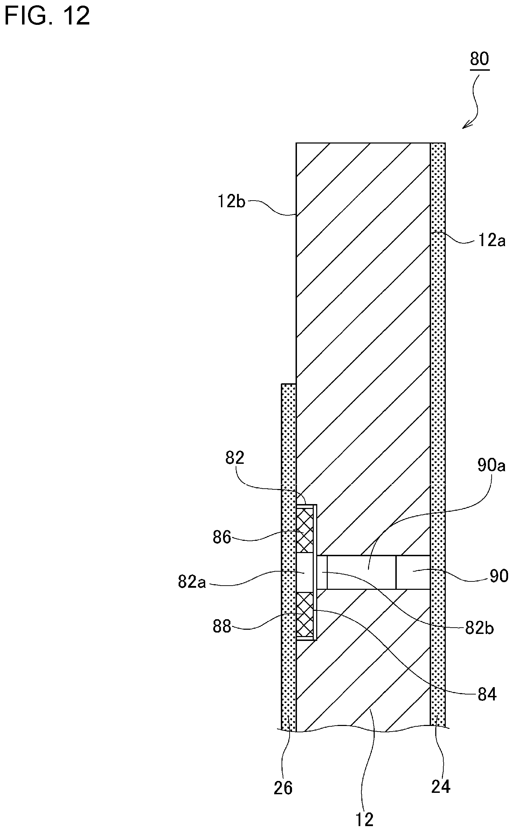

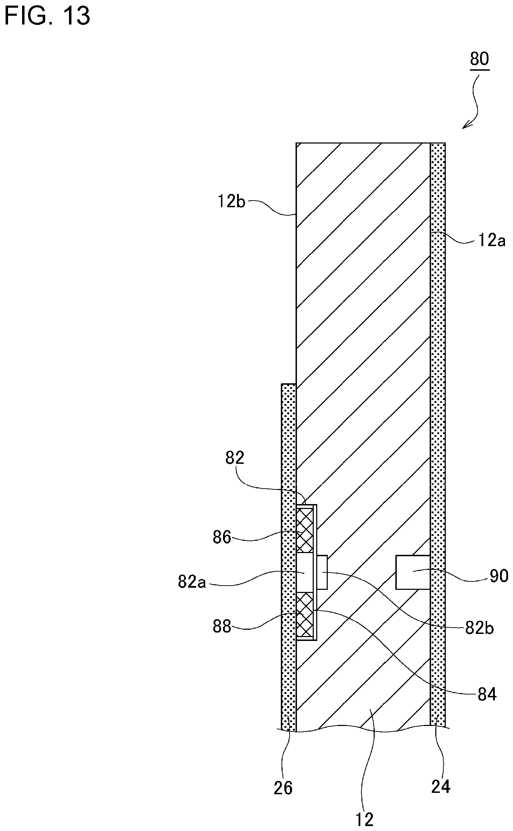

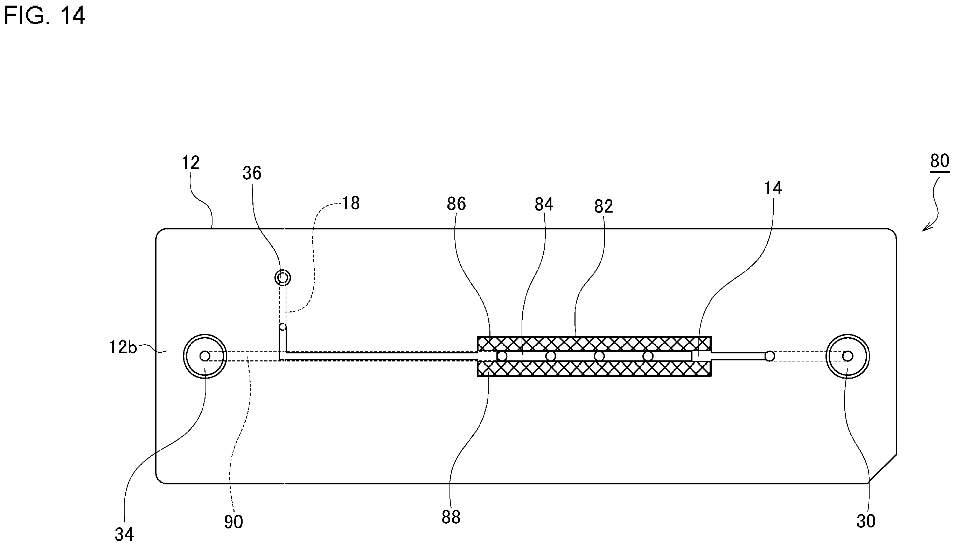

FIGS. 9A and 9B are diagrams for explaining the nucleic acid extraction container according to the second embodiment of the present invention. FIG. 10 is a cross-sectional view of the nucleic acid extraction container shown in FIG. 9A that is sectioned along line A-A. FIG. 11 is a cross-sectional view of the nucleic acid extraction container shown in FIG. 9A that is sectioned along line B-B. FIG. 12 is a cross-sectional view of the nucleic acid extraction container shown in FIG. 9A that is sectioned along line C-C. FIG. 13 is a cross-sectional view of the nucleic acid extraction container shown in FIG. 9A that is sectioned along line D-D. FIG. 14 is a plan view of a substrate provided in the nucleic acid extraction container shown in FIG. 9A. The nucleic acid extraction container 80 according to the second embodiment has a configuration different from that of the nucleic acid extraction container 10 according to the first embodiment with respect to the filter part and the second channel.

As shown in FIG. 9A, in the nucleic acid extraction container 80 according to the second embodiment, a groove-like filter part 82 is formed so as to be exposed on the upper surface 12b of the substrate 12. A rectangular hydrophilic filter 84 is installed in the filter part 82. The filter part 82 has a biological material collection area 82a on the filter 84 and a sample permeation area 82b under the filter 84. As shown in FIG. 9A, in the biological material collection area 82a, the filter 84 is fixed by placing silicone rubber 86 and silicone rubber 88 on both sides of the long sides thereof. An example of the dimensions of the filter 84 is 21 mm on the long side and 4.0 mm on the short side, and an example of the dimensions of the silicone rubber 86 and the silicone rubber 88 are 21 mm on the long side, 1.5 mm on the short side, and 0.5 mm in thickness. Therefore, the biological material collection area 82a forms a channel with a width of 1.0 mm and a depth of 0.5 mm. The nucleic acid extraction container 80 according to the second embodiment has a larger transmission area for a sample than that of the filter part 20 of the nucleic acid extraction container 10 according to the first embodiment. Therefore, the second embodiment can further increase the efficiency of the nucleic acid extraction.

As shown in FIG. 9A, the portion of the filter 84 exposed (the portion sandwiched between the silicone rubber 86 and the silicone rubber 88) is on the same straight line as the first channel 14 and the third channel 18. This facilitates replacement of air in the channels after the permeation of a sample and facilitates the movement and collection of the extraction solution.

As shown in FIG. 10, the sample permeation area 82b communicates with a first ramification channel 90a, a second ramification channel 90b, a third ramification channel 90c, and a fourth ramification channel 90d of the second channel 90. The number of branch channels can be changed appropriately according to the shape of the filter 84.

By using the nucleic acid extraction container 80 according to the second embodiment configured as described above in the same manner as the method for using the nucleic acid extraction container 10 according to the first embodiment described above, a nucleic acid in the sample can be extracted.

The above first through sixth exemplary variations can also be applied to the nucleic acid extraction container 80 according to the second embodiment.

### [Third Embodiment]

Just like the nucleic acid extraction container according to the second embodiment, a nucleic acid extraction container according to the third embodiment of the present invention also includes a substrate, a sealing film attached to the substrate, and a filter. The same components as those of the nucleic acid extraction container 80 according to the second embodiment are denoted by the same reference numerals, and redundant description will be omitted as appropriate.

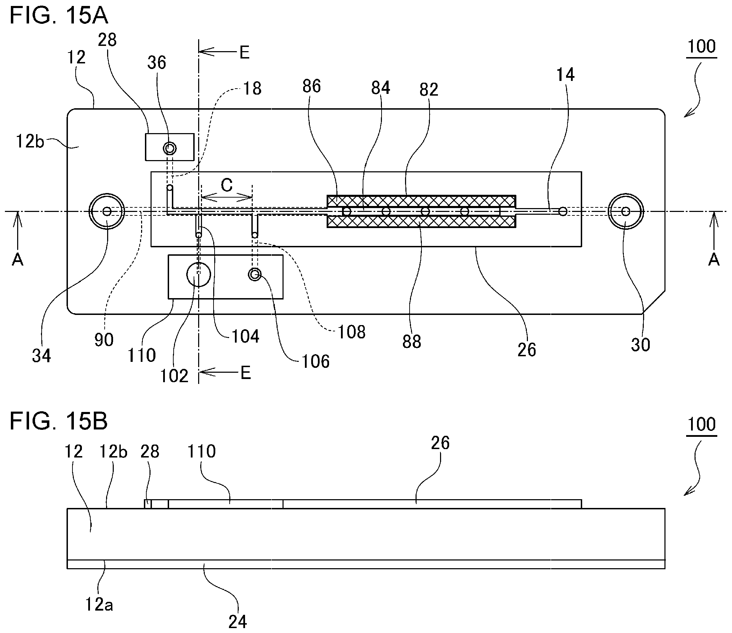

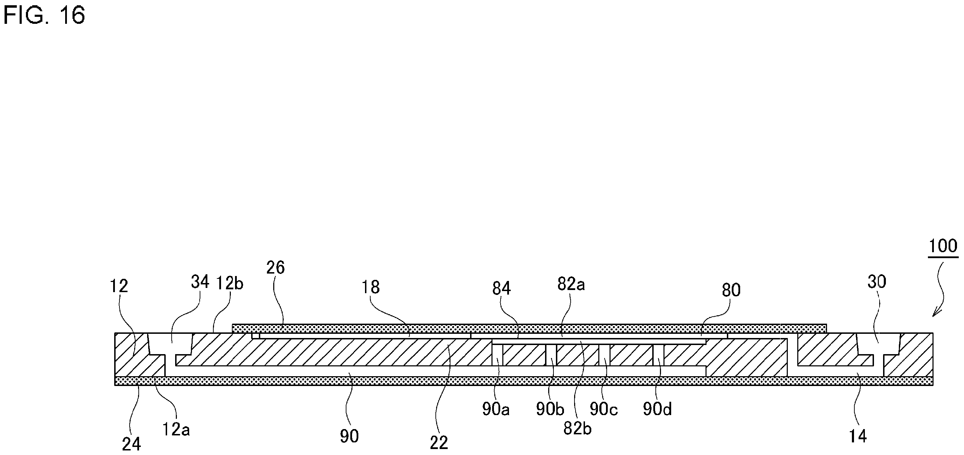

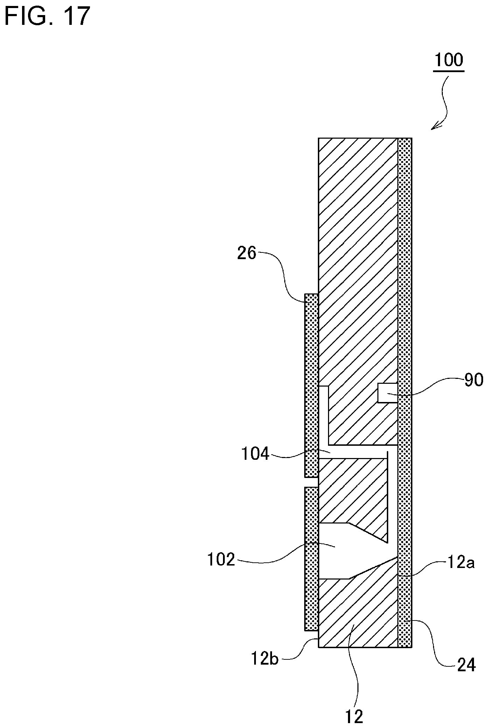

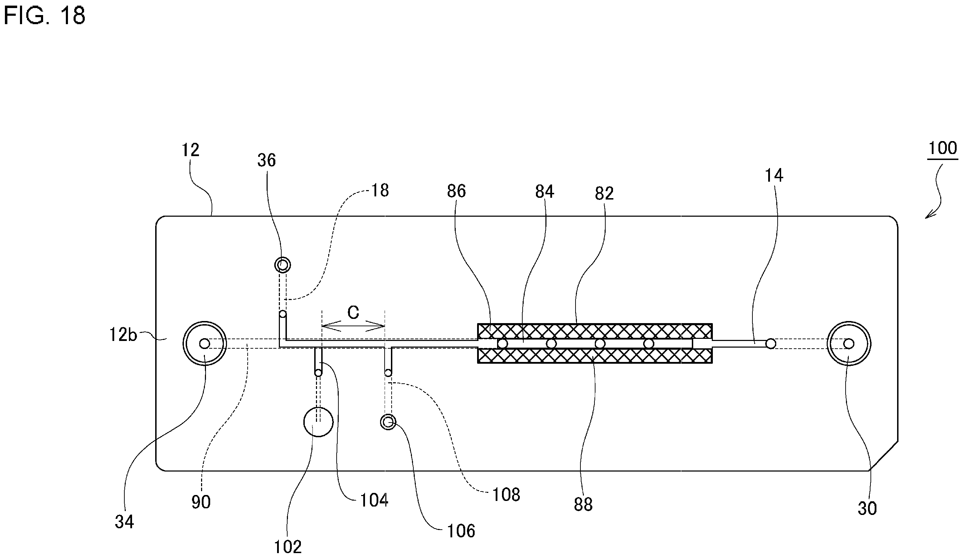

FIGS. 15A and 15B are diagrams for explaining the nucleic acid extraction container according to the third embodiment of the present invention. FIG. 16 is a cross-sectional view of the nucleic acid extraction container shown in FIG. 15A that is sectioned along line A-A. FIG. 17 is a cross-sectional view of the nucleic acid extraction container shown in FIG. 15A that is sectioned along line E-E. FIG. 18 is a plan view of a substrate provided in the nucleic acid extraction container shown in FIG. 15A. The nucleic acid extraction container 100 according to the third embodiment has a configuration different from the nucleic acid extraction container 80 according to the second embodiment in that the nucleic acid extraction container 100 is provided with a nucleic acid extraction solution outlet 102 on the upper surface 12b of the substrate 12, a second branch channel 104 communicating with the nucleic acid extraction solution outlet 102 and connected to the third channel 18, an air injection port 106 on the upper surface 12b of the substrate 12, and a third branch channel 108 communicating with the air injection port 106 and connected to the third channel 18 and in that the nucleic acid extraction solution outlet 102 and the air injection port 106 are sealed by a fourth sealing film 110. In the third embodiment, by using a region C of the third channel 18 between the connection position of the second branch channel 104 and the third channel 18 and the connection position of the third branch channel 108 and the third channel 18, a predetermined amount of a nucleic acid extraction solution can be dispensed and sent to the nucleic acid extraction solution outlet 102.

As shown in FIG. 17, the nucleic acid extraction solution outlet 102 can be shaped so as to be able to accumulate a nucleic acid extraction solution. A reagent for PCR amplification can be mixed directly into the nucleic acid extraction solution outlet 102 in which the nucleic acid extraction solution is accumulated.

An explanation will be given next regarding a method of using the nucleic acid extraction container 100 formed as described above. First, in the same manner as the method for using the nucleic acid extraction container 10 according to the first embodiment described above, a sample is passed through the filter part 82, biological materials containing a nucleic acid are collected on the filter 84, a nucleic acid extraction solution is put in the filter part 82, and a nucleic acid are extracted on the filter 84.

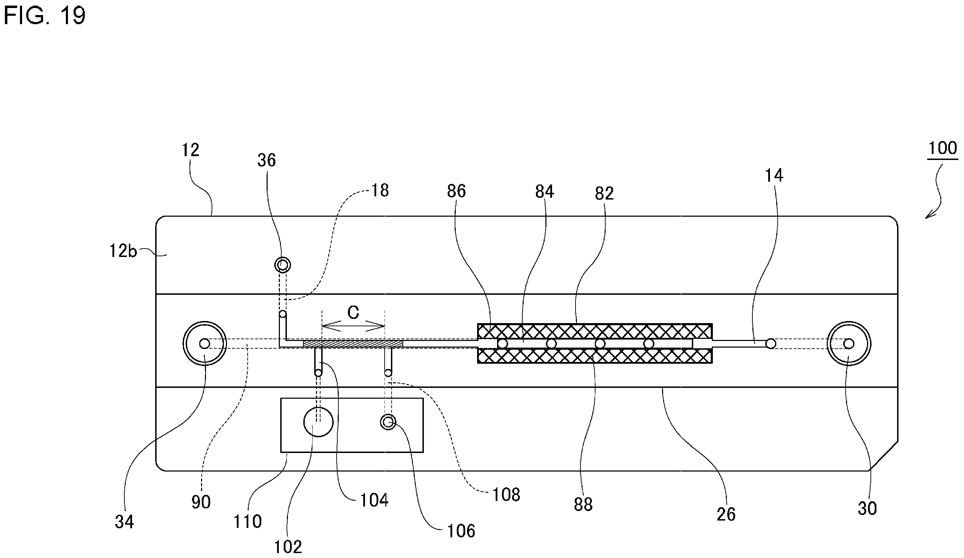

Next, the solution containing the extracted nucleic acid on the filter 84 is moved to a position including the region C of the third channel 18 by inserting a pipettor into the air communication port 36 and operating the pipettor. During the nucleic acid extraction, the third sealing film 28 is peeled off, and the air communication port 36 is open. FIG. 19 schematically shows a state where a solution containing a nucleic acid is in the region C of the third channel 18. Next, the third sealing film 28 is attached back to close the air communication port 36, and the sample injection port 30 is closed by a new sealing film (not shown). The fourth sealing film 110 is then peeled off, and the nucleic acid extraction solution outlet 102 and air injection port 106 are opened. A pipettor is inserted into the air injection port 106 so as to push air inside, only a solution containing a nucleic acid in the region C is sent to the nucleic acid extraction solution outlet 102. This dispenses the solution containing a nucleic acid. The shape of the nucleic acid extraction solution outlet 102 is such that the nucleic acid extraction solution outlet 102 can accumulate the solution as described above. Furthermore, by setting the volume of the region C to a volume that can be directly mixed with a PCR amplification reagent, the volume of the solution can be accumulated in the nucleic acid extraction solution outlet 102. In this case, the reagent for PCR amplification can be added directly to the solution accumulated in the nucleic acid extraction solution outlet 102.

The above first and fourth through sixth exemplary variations can also be applied to the nucleic acid extraction container 100 according to the third embodiment.

### [Fourth Embodiment]

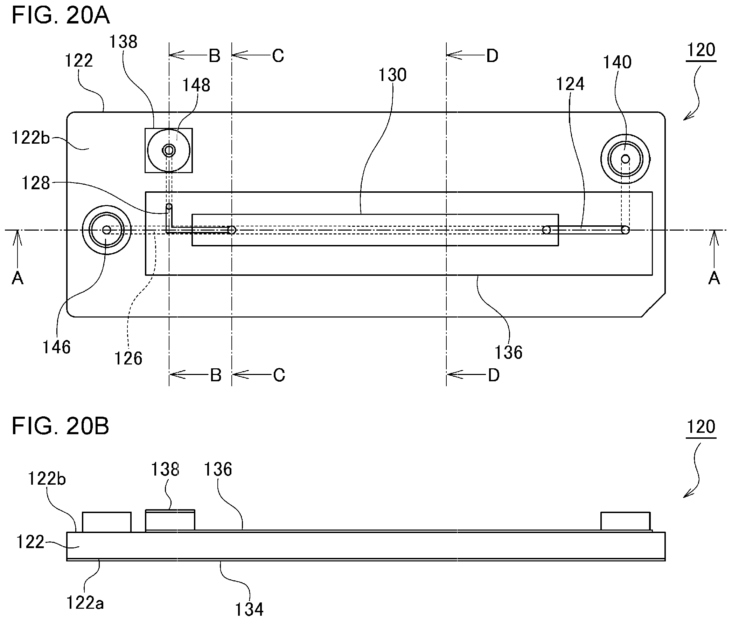

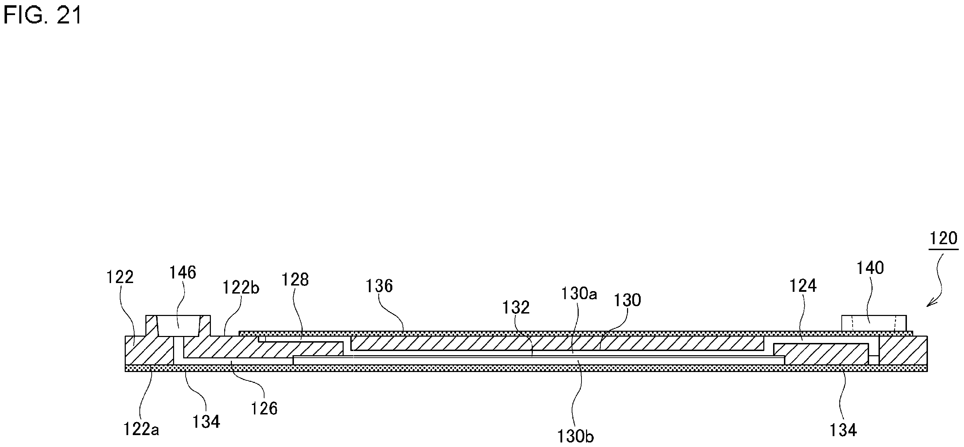

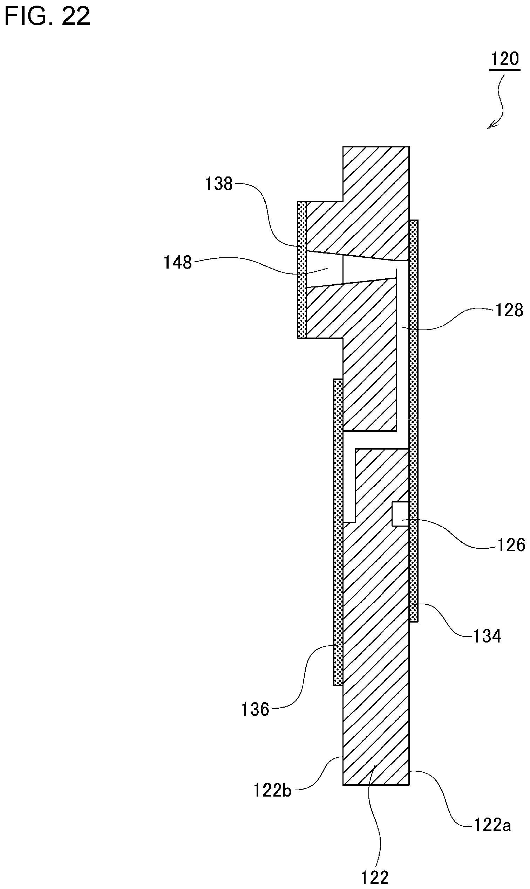

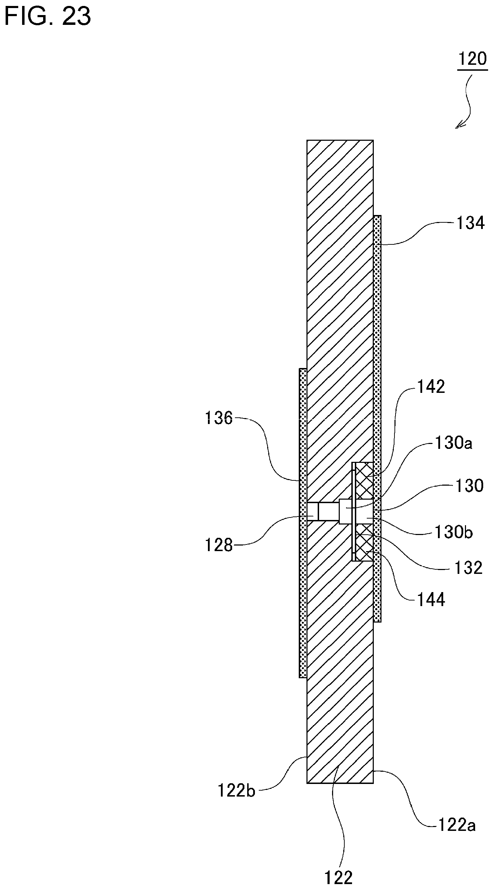

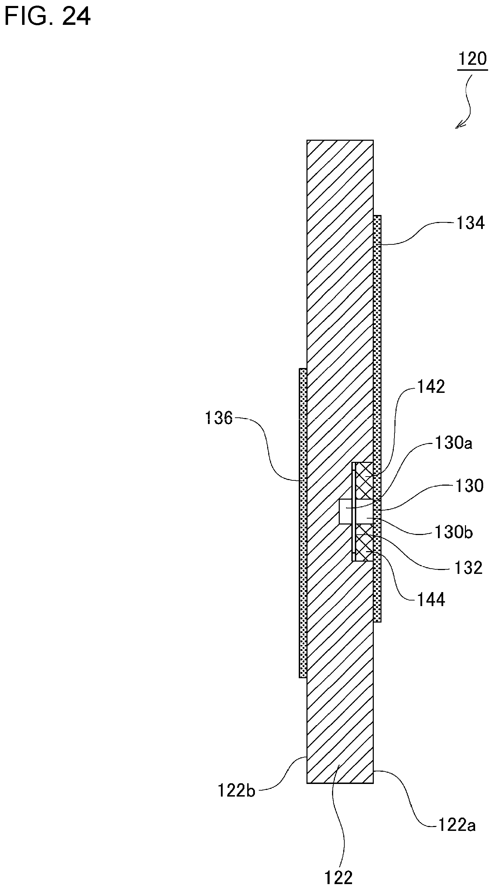

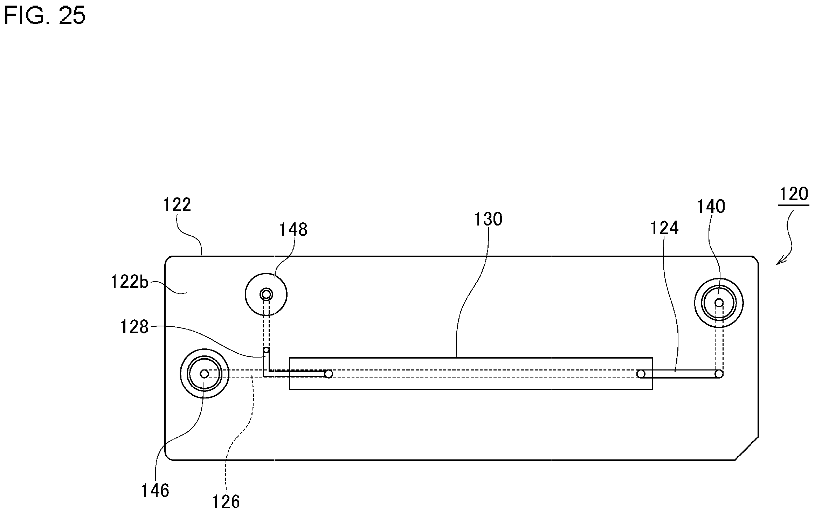

FIGS. 20A and 20B are diagrams for explaining the nucleic acid extraction container according to the fourth embodiment of the present invention. FIG. 21 is a cross-sectional view of the nucleic acid extraction container shown in FIG. 20A that is sectioned along line A-A. FIG. 22 is a cross-sectional view of the nucleic acid extraction container shown in FIG. 20A that is sectioned along line B-B. FIG. 23 is a cross-sectional view of the nucleic acid extraction container shown in FIG. 20A that is sectioned along line C-C. FIG. 24 is a cross-sectional view of the nucleic acid extraction container shown in FIG. 20A that is sectioned along line D-D. FIG. 25 is a plan view of a substrate provided in the nucleic acid extraction container shown in FIG. 20A.

A nucleic acid extraction container 120 includes: a resinous substrate 122 having a groove-like first channel 124, a second channel 126, a third channel 128, and a filter part 130 provided with a hydrophilic filter 132 that are formed on a lower surface 122a and an upper surface 122b thereof; a first sealing film 134, which is attached on the lower surface 122a of the substrate 122, for sealing the second channel 126 and the filter part 130; and two sealing films (a second sealing film 136 and a third sealing film 138) attached on the upper surface 122b of the substrate 122.

The material and dimensions of the substrate 122 are the same as those of the substrate 12 in the first embodiment. Further, the substrate 122 can be produced by injection molding, cast molding, or cutting and processing with an NC processing machine or the like in the same way as in the substrate 12 according to the first embodiment.

The composition and materials of the first sealing film 134 and the second sealing film 136 are the same as those of the first sealing film 24 and the second sealing film 26 in the first embodiment. The portions of the first sealing film 134 and the second sealing film 136 that come in contact with a channel preferably have low protein adsorption.

On the lower surface 122a and the upper surface 122b of the substrate 122, a groove-like first channel 124, a second channel 126, and a third channel 128 are formed. In the same manner as in the first embodiment, in the nucleic acid extraction container 120 according to the fourth embodiment, most parts of the first channel 124, the second channel 126, and the third channel 128 are formed in the shape of a groove exposed on the lower surface 122a and the upper surface 122b of the substrate 122. This is for allowing for inexpensive and easy molding by injection molding using a metal mold or the like. In order to seal this groove so as to make use of the groove as a channel, the first sealing film 134 is attached on the lower surface 122a of the substrate 122, and the second sealing film 136 is attached on the upper surface 122b. The dimensions of the first channel 124, the second channel 126, and the third channel 128 are the same as those of the first channel 14, the second channel 16, and the third channel 18 in the first embodiment.

The first channel 124 communicates a sample injection port 140 with a biological material collection area 130a of the filter part 130. The sample injection port 140 is formed so as to protrude on the upper surface 122b of the substrate 122. The shape of the sample injection port 140 is not limited to that shown in the figure, and just like the sample injection port 30 in the first embodiment, the sample injection port 140 may be formed to better fit a syringe or may be formed such that the sample injection port 140 can be connected to the syringe in a luer-lock manner.

The sample is introduced from the sample injection port 140 into the first channel 124. A pre-filter for removing foreign matter with a pore diameter larger than that of the filter 132 may be provided in the first channel 124.

A groove-shaped filter part 130 is formed to be exposed on the lower surface 122a of the substrate 122. The filter part 130 is sealed by the first sealing film 134. A hydrophilic filter 132 is installed in the filter part 130. As shown in FIG. 21, the filter part 130 includes a biological material collection area 130a for collecting biological materials containing a nucleic acid on the filter 132 and a sample permeation area 130b for allowing the passage of a sample having permeated through the filter 132. As explained in the first embodiment, in order to prevent the nucleic acid extraction solution from passing through the filter 132 during nucleic acid extraction, the filter 132 is desirably not in close proximity to a hydrophilic material in the sample permeation area 130b.

In the sample permeation area 130b, the filter 132 is fixed by placing silicone rubber 142 and silicone rubber 144 on both sides of the long sides thereof. Alternatively, the filter may be glued or fused to the groove surface of the lower surface 122a of the substrate 122. An example of the dimensions of the filter 132 is 40 mm on the long side and 4.0 mm on the short side, and an example of the dimensions of the silicone rubber 142 and the silicone rubber 144 are 40 mm on the long side, 1.5 mm on the short side, and 0.5 mm in thickness. Therefore, the sample permeation area 130b forms a channel with a width of 1.0 mm and a depth of 0.5 mm. The biological material collection area 130a is formed in the form of a groove on the lower surface 122a of the substrate 122 and forms a channel with the filter 132 installed in the sample permeation area 130b. An example of the dimensions of the biological material collection area 130a is 1.0 mm in width and 0.5 mm in depth. As described, by forming the filter part 130 on the lower surface 122a of the substrate 122 and fixing the filter 132 on the sample permeation area 130b side, even if foreign matter is attached to the fixture of the filter such as silicone rubber in the present embodiment or foreign matter enters during assembly of the nucleic acid extraction container, it is possible to prevent the foreign matter from getting mixed with the nucleic acid extraction solution. Further, since pressure is applied to the biological material collection area in the substrate when the sample is allowed to permeate through the filter, there is a risk that the film sealing the filter part may peel off depending on the magnitude of the pressure. However, according to the configuration of the filter part 130 in the present embodiment, the area sealing the biological material collection area 130a becomes small, and the risk of the film sealing the filter part peeling off becomes small.

The material and the pore size of the filter 132 are the same as those of the filter 22 in the first embodiment.

The second channel 126 communicates a sample discharge port 146 with the sample permeation area 130b such that the liquid that has passed through the filter 132 passes through the sample permeation area 130b and then passes through the second channel 126 so as to be discharged from the sample discharge port 146. In the present embodiment, the sample discharge port 146 is formed so as to protrude on the upper surface 122b of the substrate 122. Alternatively, the sample discharge port 146 may be formed on the lower surface 122a.

The third channel 128 communicates an air communication port 148 with the biological material collection area 130a of the filter part 130. The air communication port 148 is formed so as to protrude on the upper surface 122b of the substrate 122. As shown in FIG. 20A, a third sealing film 138 is attached to the air communication port 148. The configuration of the air communication port 148 and the configuration of the third sealing film 138 are the same as those of the air communication port 36 and the third sealing film 28 in the first embodiment.

In the same manner as in the first embodiment, the nucleic acid extraction solution can be introduced to the biological material collection area 130a of the filter part 130 from either of the sample injection port 140 and the air communication port 148. The amount of the nucleic acid extraction solution only needs to be an amount that allows contact with the entire surface of the filter 132 or an amount that allows contact with the entire surface by moving the extraction solution in contact with a portion of the filter. As in the same manner as in the first embodiment, the extraction solution brought into contact with the surface of the filter 132 can be collected from either one of the air communication port 148 and the sample injection port 140. Alternatively, the extraction solution may permeate through the filter 132 by applying pressure to the biological material collection area 130a and collected from the sample discharge port 146. The air communication port 148 may be formed so as to fit with the tip of the pipettor.

As shown in FIG. 20A, the first channel 124 and the third channel 128 preferably extend on opposite sides of each other across the biological material collection area 130a, and the first channel 124 and the third channel 128 in communication with the filter part 130 are more preferably located in a straight line. Thereby, the sample can be prevented from remaining in the first channel 124 and the filter part 130 after being passed through the filter 132.

In the nucleic acid extraction container 120, all channels are formed in a straight line. However, the form of the channels is not limited to this. For example, the channels may be formed in a so-called serpiginous manner where a turn is continuously made by combining curved portions and straight portions or may be widened in the middle. A plurality of filter parts 130 may be arranged in series. This can increase the area occupied by the filter parts 130 on the substrate 122 and allows for the collection of more biological materials.

By using the nucleic acid extraction container 120 according to the fourth embodiment configured as described above in the same manner as the method for using the nucleic acid extraction container 10 according to the first embodiment described above, a nucleic acid in the sample can be extracted.

The above first through sixth exemplary variations can also be applied to the nucleic acid extraction container 120 according to the fourth embodiment.

### [Fifth Embodiment]

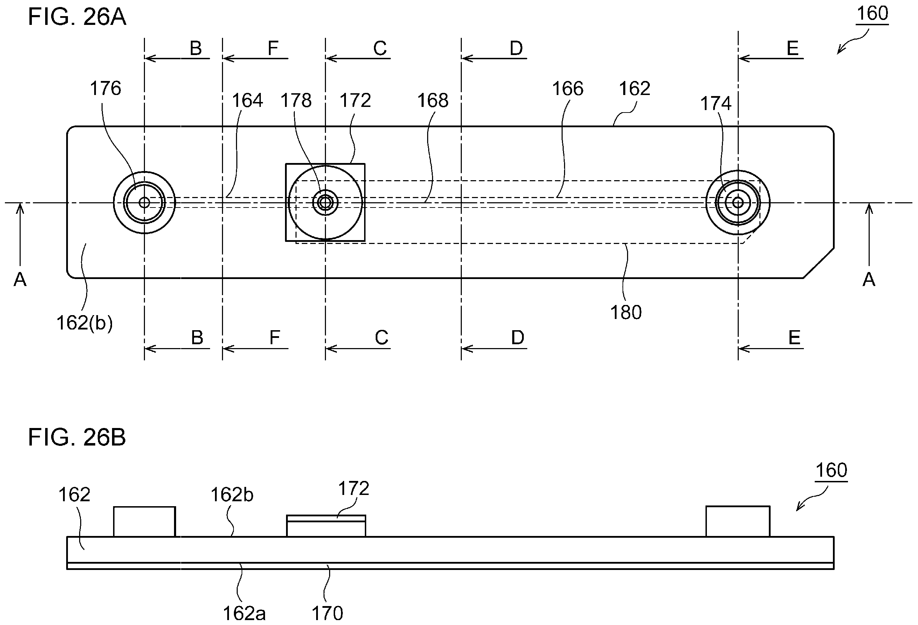

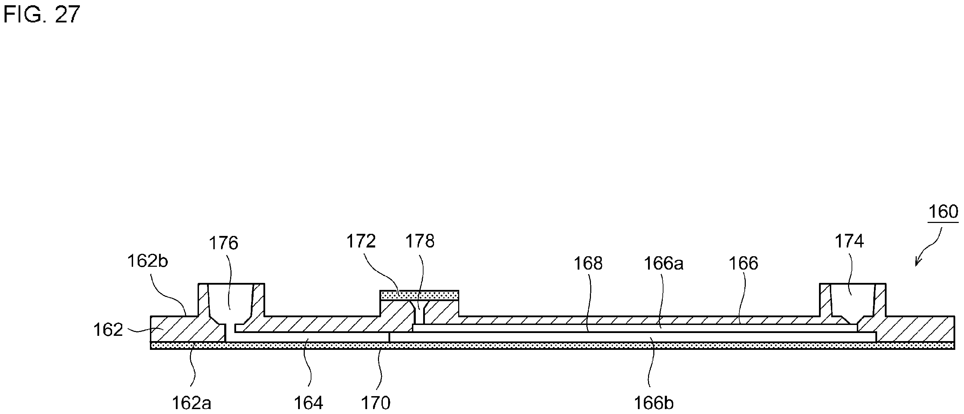

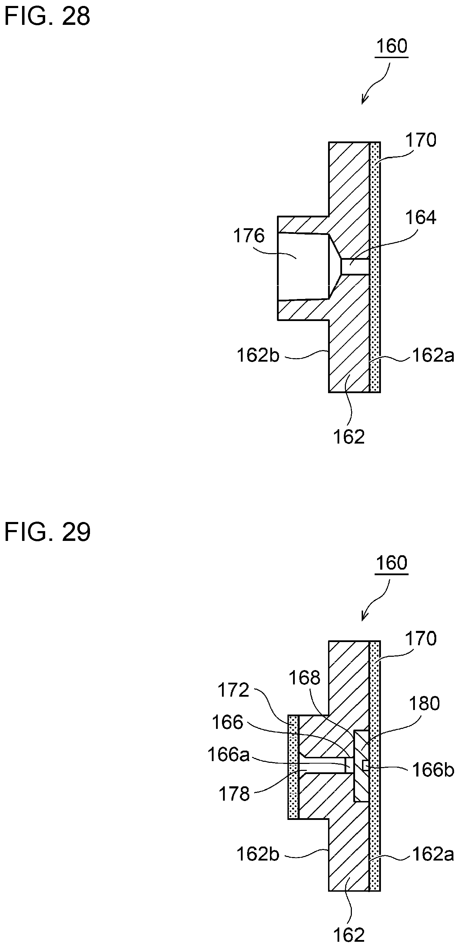

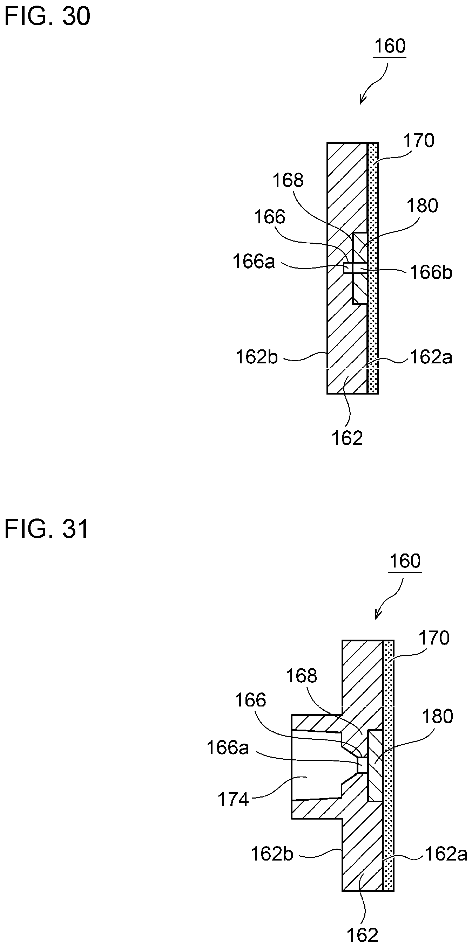

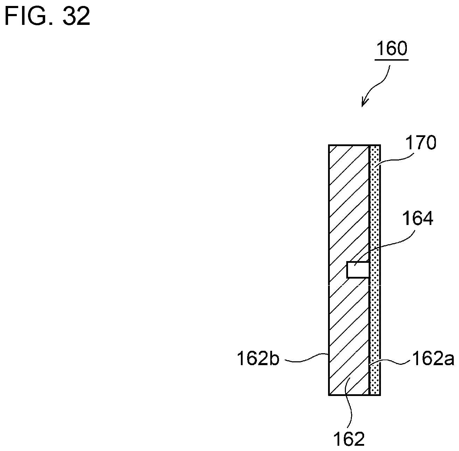

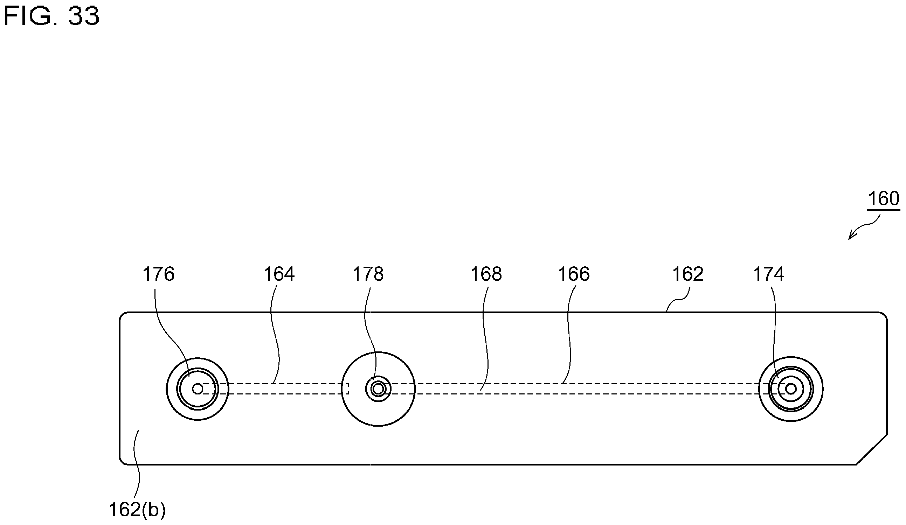

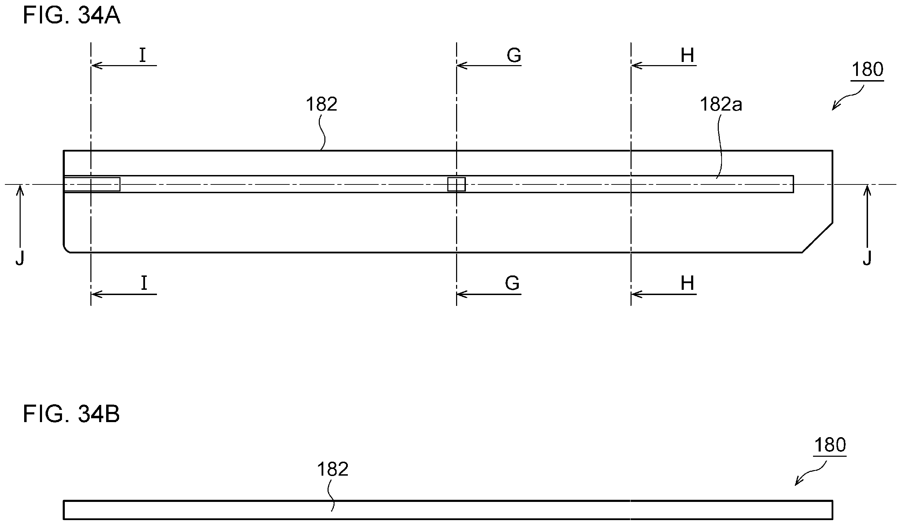

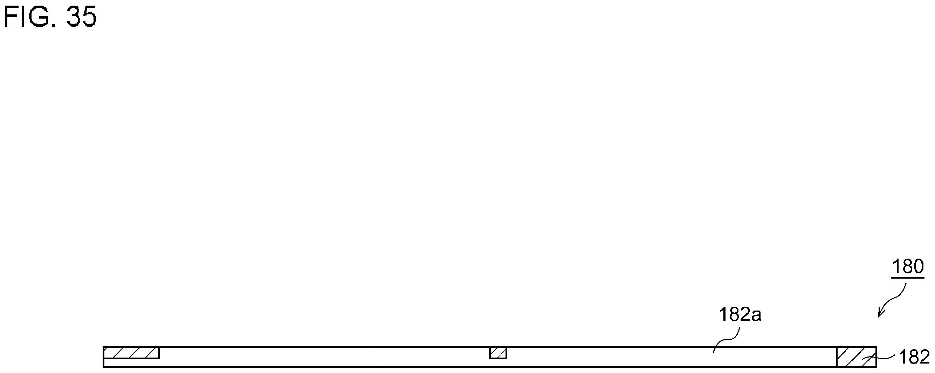

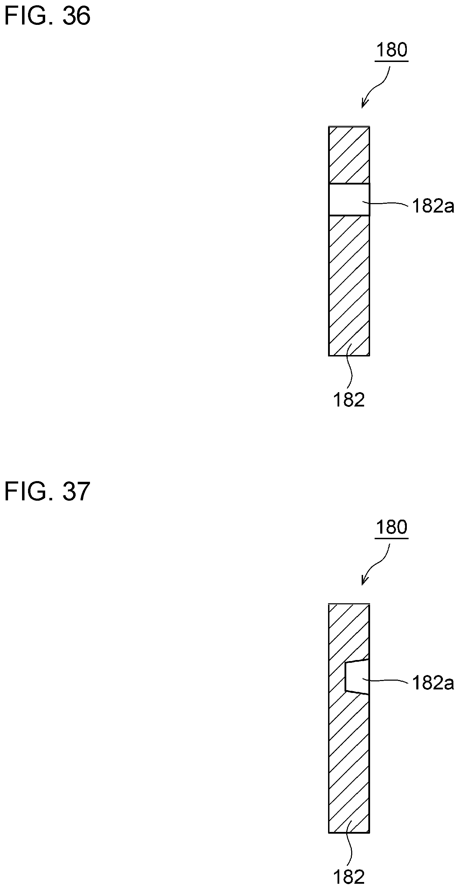

FIGS. 26A and 26B are diagrams for explaining the nucleic acid extraction container according to the fifth embodiment of the present invention. FIG. 27 is a cross-sectional view of the nucleic acid extraction container shown in FIG. 26A that is sectioned along line A-A. FIG. 28 is a cross-sectional view of the nucleic acid extraction container shown in FIG. 26A that is sectioned along line B-B. FIG. 29 is a cross-sectional view of the nucleic acid extraction container shown in FIG. 26A that is sectioned along line C-C. FIG. 30 is a cross-sectional view of the nucleic acid extraction container shown in FIG. 26A that is sectioned along line D-D. FIG. 31 is a cross-sectional view of the nucleic acid extraction container shown in FIG. 26A that is sectioned along line E-E. FIG. 32 is a cross-sectional view of the nucleic acid extraction container shown in FIG. 26A that is sectioned along line F-F. FIG. 33 is a plan view of a substrate provided in the nucleic acid extraction container shown in FIG. 26A. FIGS. 34A and 34B are diagrams for explaining a pressing plate provided in a nucleic acid extraction container according to the fifth embodiment of the present invention. FIG. 35 is a cross-sectional view of the pressing plate shown in FIG. 34A that is sectioned along line J-J. FIG. 36 is a cross-sectional view of the pressing plate shown in FIG. 34A that is sectioned along line H-H. FIG. 37 is a cross-sectional view of the pressing plate shown in FIG. 34A that is sectioned along line G-G or line I-I.

A nucleic acid extraction container 160 includes: a resinous substrate 162 having a groove-like channel 164 and a filter part 166 provided with a hydrophilic filter 168 that are formed on a lower surface 162a and an upper surface 162b thereof; a first sealing film 170, which is attached on the lower surface 162a of the substrate 162, for sealing the channel 164 and the filter part 166; and a pressing plate 180 for fixing the filter 168 to the filter part 166.

The material and dimensions of the substrate 162 are the same as those of the substrate 12 in the first embodiment. Further, the substrate 162 can be produced by injection molding, cast molding, or cutting and processing with an NC processing machine or the like in the same way as in the substrate 12 according to the first embodiment.

The composition and material of the first sealing film 170 are the same as those of the first sealing film 24 and the second sealing film 26 in the first embodiment. The portion of the first sealing film 170 that comes in contact with a channel preferably has low protein adsorption.

The groove-like channel 164 is formed on the lower surface 162a of the substrate 162. In the nucleic acid extraction container 160 according to the fifth embodiment, the channel 164 is formed in the shape of a groove exposed on the lower surface 162a of the substrate 162. This is for allowing for inexpensive and easy molding by injection molding using a metal mold or the like. In order to seal this groove so as to make use of the groove as a channel, the first sealing film 170 is attached on the lower surface 162a of the substrate 162. The dimensions of the channel 164 are the same as those of the second channel 16 in the first embodiment.

A sample injection port 174 communicates with the biological material collection area 130a of the filter part 166. The sample injection port 174 is formed so as to protrude on the upper surface 162b of the substrate 162. The shape of the sample injection port 174 is not limited to that shown in the figure, and just like the sample injection port 30 in the first embodiment, the sample injection port 174 may be formed to better fit a syringe or may be formed such that the sample injection port 174 can be connected to the syringe in a luer-lock manner.

A sample is introduced from the sample injection port 174 into the biological material collection area 166a of the filter part 166. A pre-filter for removing foreign matter with a pore diameter larger than that of the filter 168 may be provided in the sample injection port 174.

A groove-shaped filter part 166 is formed to be exposed on the lower surface 162a of the substrate 162. A hydrophilic filter 168 is installed in the filter part 166. As shown in FIG. 27, the filter part 166 includes a biological material collection area 166a for collecting biological materials containing a nucleic acid on the filter 168 and a sample permeation area 166b for allowing the passage of a sample having permeated through the filter 168. In the sample permeation area 166b, the filter 168 is fixed by installing the pressing plate 180. Alternatively, the filter 168 may be glued or fused to the groove surface of the lower surface 162a of the substrate 162. The filter part 166 is sealed by the first sealing film 170. As explained in the first embodiment, in order to prevent the nucleic acid extraction solution from passing through the filter 168 during nucleic acid extraction, the filter 168 is desirably not in close proximity to a hydrophilic material in the sample permeation area 166b.

An example of the dimensions of the filter 168 is 46 mm on the long side and 6 mm on the short side, and an example of the dimensions of the pressing plate 180 are 46 mm on the long side, 6 mm on the short side, and 1 mm in thickness. The sample permeation area 166b forms a channel with a width of 1 mm and a depth of 1 mm. The biological material collection area 166a is formed in the form of a groove on the lower surface 162a of the substrate 162 and forms a channel with the filter 168 installed in the sample permeation area 166b. An example of the dimensions of the biological material collection area 166a is 1 mm in width and 0.5 mm in depth. As described, by forming the filter part 166 on the lower surface 162a of the substrate 162 and fixing the filter 168 on the sample permeation area 166b side by the pressing plate 180, even if foreign matter is attached to the pressing plate 180 of in the present embodiment or foreign matter enters during assembly of the nucleic acid extraction container, it is possible to prevent the foreign matter from getting mixed with the nucleic acid extraction solution. Further, since pressure is applied to the biological material collection area in the substrate when the sample is allowed to permeate through the filter, there is a risk that the film sealing the filter part may peel off depending on the magnitude of the pressure. However, according to the configuration of the filter part 166 in the present embodiment, the area sealing the biological material collection area 166a becomes small, and the risk of the film sealing the filter part peeling off becomes small.

The material and the pore size of the filter 168 are the same as those of the filter 22 in the first embodiment.

As shown in Figs. 34A, 34B, and FIGS. 35 to 37, the pressing plate 180 consists of a resinous substrate 182, and a cutout 182a that constitutes the sample permeation area 166b is formed in the substrate 182. The material of the substrate 182 is the same as that of the substrate 12 in the first embodiment. The size and shape of the substrate 182 can be adjusted appropriately according to the desired size and shape of the filter part 166. The size and shape of the cutout 182a can be adjusted appropriately according to the desired size and shape of the sample permeation area 166b. Further, the substrate 182 can be produced by injection molding, cast molding, or cutting and processing with an NC processing machine or the like in the same way as in the substrate 12 according to the first embodiment.

The channel 164 communicates a sample discharge port 176 with the sample permeation area 166b such that the liquid that has passed through the filter 168 passes through the sample permeation area 166b and then passes through the channel 164 so as to be discharged from the sample discharge port 176. In the present embodiment, the sample discharge port 176 is formed so as to protrude on the upper surface 162b of the substrate 162. Alternatively, the sample discharge port 176 may be formed on the lower surface 162a.

An air communication port 178 communicates with the biological material collection area 166a of the filter part 166. The air communication port 178 is formed so as to protrude on the upper surface 162b of the substrate 162. As shown in FIG. 26A, a second sealing film 172 is attached to the air communication port 178. The configuration of the air communication port 178 and the configuration of the second sealing film 172 are the same as those of the air communication port 36 and the third sealing film 28 in the first embodiment.

In the same manner as in the first embodiment, the nucleic acid extraction solution can be introduced to the biological material collection area 166a of the filter part 166 from either of the sample injection port 174 and the air communication port 178. The amount of the nucleic acid extraction solution only needs to be an amount that allows contact with the entire surface of the filter 168 or an amount that allows contact with the entire surface by moving the extraction solution in contact with a portion of the filter. As in the same manner as in the first embodiment, the extraction solution brought into contact with the surface of the filter 168 can be collected from either one of the air communication port 178 and the sample injection port 174. Alternatively, the extraction solution may permeate through the filter 168 by applying pressure to the biological material collection area 166a and collected from the sample discharge port 176. The air communication port 178 may be formed so as to fit with the tip of the pipettor.

As shown in FIGS. 26A and 27, the sample injection port 174 and the air communication port 178 directly communicates with the biological material collection area 166a. Furthermore, there are fewer sealing films compared with the first through fourth embodiments. Therefore, the nucleic acid extraction container according to the fifth embodiment can be manufactured at a lower cost than the first through fourth embodiments.

As shown in FIGS. 26A and 27, the sample injection port 174 and the air communication port 178 are preferably arranged on opposite sides of each other across the biological material collection area 166a. This can ensure that the sample does not remain in the filter part 166 after the sample is passed through the filter 168.

In the nucleic acid extraction container 160, channels are formed in a straight line. However, the form of the channels is not limited to this. For example, the channels may be formed in a so-called serpiginous manner where a turn is continuously made by combining curved portions and straight portions or may be widened in the middle. A plurality of filter parts 166 may be arranged in series. This can increase the area occupied by the filter parts 166 on the substrate 162 and allows for the collection of more biological materials.

By using the nucleic acid extraction container 160 according to the fifth embodiment configured as described above in the same manner as the method for using the nucleic acid extraction container 10 according to the first embodiment described above, a nucleic acid in the sample can be extracted.

The above fifth and sixth exemplary variations can also be applied to the nucleic acid extraction container 160 according to the fifth embodiment.

### [Exemplary Embodiments]

Hereinafter, exemplary embodiments of the present invention will be explained. However, these exemplary embodiments are merely examples for suitably explaining the present invention and do not limit the present invention in any way.

In the present exemplary embodiment, the method using the nucleic acid extraction solution according to the embodiment is compared for the analysis of environmental DNA with the conventional academic standard method. Environmental DNA (eDNA) refers to DNA released into the environment and derived from organisms living there. More specifically, various organisms living in the environment constantly release their DNA into the surroundings. For example, in the case of animals, their DNA is released through their skin, body hair, excrement, dead bodies, etc. By detecting or quantifying environmental DNA included in the environment, it is possible to identify the species of organisms living in that environment and to understand the biodiversity of that environment. The type of DNA to be analyzed is often mitochondrial DNA from the viewpoint of stability. In the academic standard method, several hundred mL to 1 L of sample water is filtered, and DNA is extracted from particles containing environmental DNA collected through the filtering so as to achieve a state where the DNA is extracted into a 200 µL solution in the end. Then, a portion of this liquid (about 2 µL) is used for detection by PCR to see whether or not the organism to be measured exists or to what extent the organism to be measured exists.

The reagents used in the PCR in the present exemplary embodiment are listed in Table 1 below.

**[Table 1]**

| | Final Concentration | | Remarks |
|---|---|---|---|
| | PCR1100 | StepOne | Remarks |
| Enzyme | 0.1 U/uL | 0.03 U/uL | KAPA 3G Plant (manufactured by Nippon Genetics Co. ,Ltd.) |
| Primer F | 900 nM | 900 nM | The primer with the following sequence |
| Primer R | 900 nM | 900 nM | The primer with the following sequence |
| Probe | 400 nM | 150 nM | The probe with the following sequence |
| Additional Mg | 2 mM | | Came with KAPA 3G Plant |
| Solution B | | 1.4 % | Came with Easy Extraction Kit Version 2 by Kaneka Corporation |
| Buffer + | | | According to KAPA 3G |
| Water | | | Plant manual, buffer and water were blended so that concentration of attached buffer is diluted to 1/2 of total reagent. |

The sequences of the forward primer (primer F), the reverse primer (primer R) and the probe used in PCR are as follows.
Oncorhynchus mykiss Primer F: 5'-AGTCTCTCCCTGTATATCGTC-3' (sequence number 1)
Oncorhynchus mykiss Primer R: 5'-GATTTTAGTTCATGAAGTTGCGAGAGAGTA-3' (sequence number 2)
Oncorhynchus mykiss Probe: 5'-CCAACAACTCTTTAACCATC-3' (sequence number 3)

The 5' side of the probe was labeled with FAM, and the 3' side was labeled with [NFQ]-[MGB].
(Reference: Wilcox, T.M., Carim, K.J., McKelvey, K.S., Young, M.K., & Schwartz, M.K. 2015 (4 Nov.). The dual challenges of generality and specificity when developing environmental DNA markers for species and subspecies of Oncorhynchus. PLoS ONE, 10(11) e0142008. doi:10.1371/journal.pone.0142008.)
Cyprinus carpio Primer F: 5'-GGTGGGTTCTCAGTAGACAATGC-3' (sequence number 4)
Cyprinus carpio Primer R: 5'-GGCGGCAATAACAAATGGTAGT-3' (sequence number 5)
Cyprinus carpio Probe: 5'-CACTAACACG ATTCTTCCGCA TTCCACTTCC-3'' (sequence number 6)

The 5' side of the probe was labeled with FAM, and the 3' side was labeled with TAMRA.
(Reference: Takahara, T., Minamoto, T., Yamanaka, H., Doi, H. & Kawabata, Z. 2012. Estimation of fish biomass using environmental DNA, PLoS ONE, 7: e35868.)
Katsuwonus pelamis Primer F: 5'-TACCCCTGACGTAGAATCAGCC-3' (sequence number 7)
Katsuwonus pelamis Primer R: 5'-GGGCCAATATGGGGAGTAAATGCAG-3' (sequence number 8)
Katsuwonus pelamis Probe; 5'-TGCCGAGACGTAAACTTCGG-3' (sequence number 9)

The 5' side of the probe was labeled with Cy5, and the 3' side was labeled with BHQ3.
(Reference: Lin, W.-F. & Hwang, D.-F. 2008. Application of species-specific PCR for the identification of dried Katsuwonus pelamis product (Katsuobushi). Food Chemistry 106: 390-396.)

The conditions for PCR amplification reaction were as follows. 95 degrees Celsius for 15 seconds, followed by repetition of 60 degrees Celsius for 10 seconds and 95 degrees Celsius for 3.5 seconds.

### (First Exemplary Embodiment)

The nucleic acid extraction container 10 shown in FIGS. 1A and 1B was prepared. The nucleic acid extraction container 10 has the shape of a plate of 26*75*4 mm, and the material thereof is cyclo-olefin polymer (COP). The channels, the filter part, and the air communication port were sealed by applying polyolefin micro sealing tape (9795) from 3M Company. The size of the first channel 14 and the size of the second channel 16 are 1.0 mm in width and 1.0 mm in depth, and the size of the third channel 18 is 0.6 mm in width and 0.6 mm in depth. The size of the hydrophilic filter is ϕ4 mm. The filter 22 is fixed by an O-ring 32 of ϕ4*ϕ2. The material of the filter 22 is polyvinylidene fluoride (PVDF), and the pore size is 0.45 um. The effective surface of the filter 22 is ϕ3 mm.

Using this nucleic acid extraction container, an extraction solution from which DNA was extracted was obtained by the following procedure.
(1) A 10-mL syringe was inserted into the sample injection port 30, and 2 mL of the sample was poured from here. At this time, a tube was inserted into the sample discharge port 34, and a discharged solution was discarded.
(2) The syringe was pulled out once, and after sucking 10 mL of air, the syringe was inserted into the sample injection port 30 again, and 10 mL of air was poured into the channels. Thereby, the sample in the first channel 14 and the second channel 16 was discharged.
(3) The sealing film of the air communication port 36 was peeled off, and 5 µL of the DNA extraction solution was injected from here with a pipettor. While visually checking the position, the DNA extraction solution was pushed into the filter part 20 and then left for one minute.
(4) The pipettor was inserted into the air communication port 36 so as to suck up the extraction solution.

The sample was diluted water of a suspension of raw Katsuwonus pelamis meat suspended in pure water. As the DNA extraction solution, 200 mM of Na2CO3 solution was used.

1.6 µL of the extraction solution obtained using the nucleic acid extraction container 10 was mixed with 14.4 µL of the PCR amplification reagent and amplified by PCR1100 manufactured by Nippon Sheet Glass Company, and a cycle threshold (Ct value) of 38.9 was obtained. The filtering and the extraction took about three minutes. In the present exemplary embodiment, the extraction solution was directly mixed with the PCR amplification reagent. Alternatively, the extraction solution may be mixed with the PCR reagent after being mixed with a neutralizing solution or the like.

### (First Comparative Example)

After filtering 50 mL of the same sample as that in the first exemplary embodiment in accordance with "Environmental DNA Sampling and Experiment Manual (ver2.1)" (The eDNA Society, Internet <URL: http://ednasociety.org/eDNA_manual_ver2_1_3.pdf>) using a cartridge-type filter (Sterivex 0.45 µm, manufactured by Merck), DNA was extracted using Dneasy Blood and Tissue kit from QIAGEN N.V. 1.6 µL of the extraction solution obtained was mixed with 14.4 µL of the PCR amplification reagent and amplified using the same mobile PCR device (product name: PicoGene PCR1100) as that in the first exemplary embodiment, and a Ct value of 40.1 was obtained. The filtering and the extraction took about two hours.

The results of the first exemplary embodiment and the first comparative example are shown in Table 2 below.

**[Table 2]**

| | Sample | Extraction Method | Ct Value |
|---|---|---|---|
| First Exemplary Embodiment | 2 mL of diluted water of suspension of raw Katsuwonus pelamis meat | Na₂CO₃ 200 mM 5 µL | 38.9 |
| First Comparative Example | 50 mL of water in first exemplary embodiment | Follow Manual by The eDNA Society | 40.1 |

The amount of the sample in the first exemplary embodiment is 1/10 or less of that in the first comparative example. Further, in the first exemplary embodiment, the process from filtering to extraction is simpler than that in the first comparative example, and the time required for the process is about three minutes compared to two hours in the first comparative example. Further, as shown in Table 2, the Ct value obtained in the first exemplary embodiment is almost the same as that obtained in the first comparative example. This indicates that detection sensitivity equivalent to that in the first comparative example can be obtained in the first exemplary embodiment even though the amount of sample is 1/10 of that in the first comparative example. The number of consumables used in the first exemplary embodiment is small, and the nucleic acid extraction container 10 used for the extraction has a simple structure and can be manufactured at low cost. In nucleic acid measurement, generally, many items are disposable in order to avoid contamination called carryover, so it is important to reduce the cost of the consumables. Furthermore, in the first exemplary embodiment, since the amount of the sample is small, it is easy to send the sample water to the testing company. In addition, in the first exemplary embodiment, no special equipment is required and the work procedures are few and simple, making it easy for anyone to perform extraction even on site. In addition, in the first exemplary embodiment, the risk of contamination is reduced because filtering and extraction can be performed in a closed space.

### (Second Exemplary Embodiment)

The nucleic acid extraction container 80 shown in FIGS. 9A and 9B was prepared. The nucleic acid extraction container 80 has the shape of a plate of 26*75*4 mm, and the material thereof is cyclo-olefin polymer (COP). The channels, the filter part, and the air communication port were sealed by applying polyolefin micro sealing tape (9795) from 3M Company. The size of the first channel 14 and the size of the second channel 16 are 1.0 mm in width and 1.0 mm in depth, and the size of the third channel 18 is 0.6 mm in width and 0.6 mm in depth. The size of the filter 84 is 21 mm in length and 4 mm in width. The filter 84 was fixed by pressing both sides of the filter 84 from above with the silicone rubber 86 and the silicone rubber 88 both having a width of 1.5 mm and a thickness of 0.5 mm. The material of the filter 84 is polyvinylidene fluoride (PVDF), and the pore size is 0.45 um. The effective surface of the filter 84 is 20 mm².

Using this nucleic acid extraction container 80, an extraction solution from which DNA was extracted was obtained by the following procedure.
(1) A 10-mL syringe was inserted into the sample injection port 30, and 10 mL or 5 mL of the sample was poured from here. At this time, a tube was inserted into the sample discharge port 34, and a discharged solution was discarded.
(2) The syringe was pulled out once, and after sucking 10 mL of air, the syringe was inserted into the sample injection port 30 again, and 10 mL of air was poured into the first channel and the second channel.
(3) The third sealing film 28 of the air communication port 36 was peeled off, and 5 µL of the DNA extraction solution was injected from here with a pipettor. The DNA extraction solution was pushed into the filter part 82. Then, while visually checking the position, the DNA extraction solution was reciprocated using a pipettor for one minute such that the DNA extraction solution touched the entire effective surface of the filter 84.
(4) The pipettor was inserted into the air communication port 36 so as to suck up the extraction solution.

The samples were 10 mL of Oncorhynchus mykiss tank water diluted 100-fold with pure water (Exemplary Embodiment 2-1) and 5 mL of water from a pond in Tsukuba City where Cyprinus carpio lived (Exemplary Embodiment 2-2). As the DNA extraction solution, 200 mM of Na2CO3 solution (Exemplary Embodiment 2-1) and Easy Extraction Kit Version 2 (Exemplary Embodiment 2-2) from Kaneka Corporation were used.

1.6 µL of the obtained DNA extraction solution was taken out, then directly mixed with 14.4 µL of the PCR amplification reagent, and amplified using the same mobile PCR device as that in the first exemplary embodiment. The results shown in Table 3 below were obtained. The extraction solution was directly mixed with the PCR amplification reagent at this time. Alternatively, the extraction solution may be mixed with the PCR reagent after being mixed with a neutralizing solution or the like. The filtering and the extraction took about three minutes.

**[Table 3]**

| | Sample | Extraction Solution | Ct Value |
|---|---|---|---|
| Exemplary Embodiment 2-1 | 10 mL of Oncorhynchus mykiss tank water diluted 100-fold with pure water | Na₂CO₃ 200 mM 5 µL | 30.3 |
| | | | 29.7 |
| Exemplary Embodiment 2-2 | 5 mL of water from a pond in Tsukuba City where Cyprinus carpio lived | 5 µL of A solution of Easy Extraction Kit by Kaneka Corporation | 35.8 |
| | | | 35.7 |

In the DNA extraction in the present exemplary embodiment, the DNA extraction solution is in contact with only about half of the filter. However, it was found that DNA trapped in the entire area of the filter was able to be extracted by reciprocating the DNA extraction solution. This means that the sensitivity can be further increased by increasing the length of the filter part and increasing the amount of sample transmission.

Usually, extraction of a nucleic acid is performed by immersing an object to be extracted (e.g., mouse tail, plant, etc.) in an extraction solution so as to dissolve the object and extracting the nucleic acid. In the present exemplary embodiment, it was found that without immersion in an extraction solution, extraction was able to be performed simply by allowing the extraction solution to flow over a filter on which biological materials were collected. Being able to perform extraction simply by the flowing means that the amount of the extraction solution may be smaller than that used when immersing the entirety, and this leads to an increase in the concentration of a nucleic acid in the extraction solution.

### (Second Comparative Example)

In the same manner as in the first comparative example, DNA was extracted from 100 mL of the same sample as in Exemplary Embodiment 2-1 (Comparative Example 2-1) and from 50 mL of the same sample as in Exemplary Embodiment 2-2 (Comparative Example 2-2). 1.6 µL of the obtained extraction solution was mixed with 14.4 µL of the PCR amplification reagent and amplified using the same mobile PCR device as that in the first exemplary embodiment. The results shown in Table 4 below were obtained. The filtering and the extraction took about two hours.

**[Table 4]**

| | Sample | Extraction Method | Ct Value |
|---|---|---|---|
| Comparative Example 2-1 | 100 mL of water in Exemplary Embodiment 2-1 | Follow Manual by The eDNA Society | 32.0 |
| | | | 30.5 |
| Comparative Example 2-2 | 50 mL of water in Exemplary Embodiment 2-2 | Follow Manual by The eDNA Society | 35.3 |
| | | | 35.4 |

In the second exemplary embodiment, even though the same samples as those in the second comparative example were used, almost the same Ct values were obtained using onetenth of the sample amounts, respectively, and the same amounts of DNA were able to be detected. That is, the detection sensitivity in the second exemplary embodiment is equivalent to that in the second comparative example, and as in the first exemplary embodiment, the second exemplary embodiment has the advantages of easy shipping of samples to a testing company, a simple extraction procedure, and low contamination.

### (Third Exemplary Embodiment)

The nucleic acid extraction container 100 shown in FIGS. 15A and 15B was prepared. The nucleic acid extraction container 100 has the shape of a plate of 26*75*4 mm, and the material thereof is cyclo-olefin polymer (COP). The channels, the filter part, and the injection port were sealed by applying polyolefin micro sealing tape (9795) from 3M Company. The sizes of the first channel 14 and the second channel 16 are 1.0 mm in width and 1.0 mm in depth. The size of the third channel 18 is 0.6 mm in width and 0.6 mm in depth. The sizes of the second branch channel 104 and the third branch channel 108 are the same as that of the third channel 18. The size of the filter 84 is 21 mm in length and 4 mm in width. The filter 84 was fixed by pressing both sides of the filter 84 from above with the silicone rubber 86 and the silicone rubber 88 both having a width of 1.5 mm and a thickness of 0.5 mm. The material of the filter 84 is polyvinylidene fluoride (PVDF), and the pore size is 0.45 um. The effective surface of the filter 84 is 20 mm².

Using this nucleic acid extraction container 100, an extraction solution from which DNA was extracted was obtained by the following procedure. The sample is 10 mL of the water according to Exemplary Embodiment 2-1.
(1) A 10-mL syringe was inserted into the sample injection port 30, and 10 mL of the sample was poured from here. At this time, a tube was inserted into the discharge port, and a discharged solution was discarded.
(2) The syringe was pulled out once, and after sucking 10 mL of air, the syringe was inserted into the sample injection port 30 again, and 10 mL of air was poured into the channels.
(3) The third sealing film 28 was peeled off, and 5 µL of the extraction solution was injected from the air communication port 36 with a pipettor so as to push the extraction solution into the filter 84. After that, while visually checking the position, the extraction solution was reciprocated using a pipettor for one minute such that the extraction solution touched the entire effective surface of the filter 84, and the extraction solution was stopped at a portion including the region C of the third channel 18 in the end.
(4) The third sealing film was attached back so as to seal the air communication port 36, and the sample injection port 30 was sealed by a new sealing film. Then, the fourth sealing film 110 was peeled off, and a pipettor was inserted into the air injection port 106 so as to push air inside. Thereby, the extraction solution was collected in a reservoir of the nucleic acid extraction solution outlet 102.

The extraction solution collected in the reservoir was dispensed into 1.6 µL. To this, 14.4 µL of the PCR amplification reagent was added and mixed. DNA in this mixture was amplified using the same mobile PCR device as in the first exemplary embodiment, and a Ct value of 30.2 was obtained. The filtering and the extraction took about three minutes. In the present exemplary embodiment, the extraction solution was directly mixed with the PCR amplification reagent. Alternatively, the extraction solution may be mixed with the PCR reagent after being mixed with a neutralizing solution or the like. Compared to the second exemplary embodiment, dispensing of 1.6 µL is no longer necessary, and a tube for mixing with the PCR amplification reagent is also no longer necessary. Thus, it is easier to move from extraction to PCR amplification.

**[Table 5]**

| | Sample | Extraction Solution | Ct Value |
|---|---|---|---|
| Third Exemplary Embodiment | 10 mL of water according to Exemplary Embodiment 2-1 | Na₂CO₃ 200 mM 5 µL | 30.2 |

### (Fourth Embodiment)

The nucleic acid extraction container 160 shown in FIGS. 26A and 26B was prepared. The nucleic acid extraction container 160 has the shape of a plate of 26*75*4 mm, and the material thereof is cyclo-olefin polymer (COP). The channels, the filter part, and the injection port were sealed by applying polyolefin micro sealing tape (9795) from 3M Company. The size of the channel 164 is 1.0 mm in width and 1.0 mm in depth. The sample permeation area 166b serves as a channel with a width of 1 mm and a depth of 1 mm. The biological material collection area 166a serves as a channel of 1 mm in width and 0.5 mm in depth. The size of the filter 168 is 46 mm in length and 6 mm in width. By placing the pressing plate 180 shown in FIG. 34 in the nucleic acid extraction container 160, the filter 168 was fixed. The pressing plate 180 has a plate shape of 6*46*1 mm. The material of the filter 168 is polyvinylidene fluoride (PVDF), and the pore size is 0.65 um. The effective surface of the filter 168 is 40 mm². The volume of the biological material collection area 166a is 20 µL.

Using this nucleic acid extraction container 160, an extraction solution from which DNA was extracted was obtained by the following procedure.
(1) A 10-mL syringe was inserted into the sample injection port 174, and 10 mL of the sample was poured from here. At this time, a tube was inserted into the discharge port, and a discharged solution was discarded.
(2) The syringe was pulled out once, and after sucking 10 mL of air, the syringe was inserted into the sample injection port 174 again, and 10 mL of air was poured into the channels.
(3) The second sealing film 172 was peeled off, and a predetermined amount of the extraction solution was injected from the air communication port 178 with a pipettor so as to push the extraction solution into the filter 168. After that, while visually checking the position, the extraction solution was reciprocated using a pipettor for two minutes such that the extraction solution touched the entire effective surface of the filter 168.
(4) Using the same pipettor, the extraction solution was collected from the air communication port 178.

The samples were 10 mL of Oncorhynchus mykiss tank water diluted 50-fold with pure water. As the DNA extraction solution, Easy Extraction Kit Version 2 from Kaneka Corporation was used.

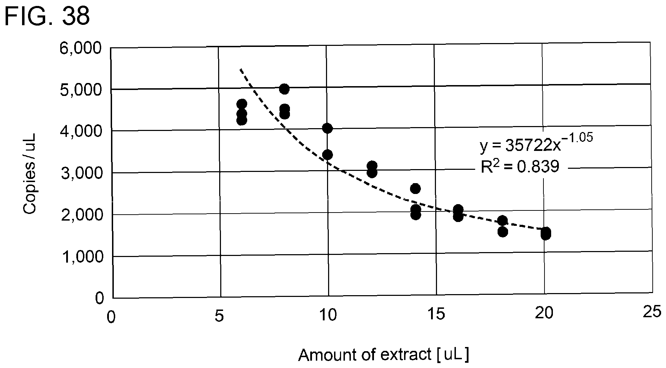

1.5 µL of the obtained DNA extraction solution was taken out, then directly mixed with 13.5 µL of the PCR amplification reagent, and amplified using StepOne Plus Real Time PCR System (Applied Biosystems). The relationship between the volume of the extraction solution and the DNA concentration (copies/uL) is shown in FIG. 38. The DNA concentration was obtained by conversion from the resulting Ct value from a calibration curve prepared during PCR.

Based on FIG. 38, it can be found that the DNA concentration in the extraction solution increases when extraction is performed with a small amount of extraction solution.

Described above is an explanation of the present invention based on the embodiments. The embodiment is intended to be illustrative only, and it will be obvious to those skilled in the art that various modifications to constituting elements and processes could be developed and that such modifications are also within the scope of the present invention.

### [INDUSTRIAL APPLICABILITY]

The present invention is applicable to a nucleic acid extraction container and a nucleic acid extraction method.

### [REFERENCE SIGNS LIST]

10 nucleic acid extraction container, 14 first channel, 16 second channel, 18 third channel, 20 filter part, 20a biological material collection area, 20b sample permeation area, 22 filter, 30 sample injection port, 34
sample discharge port, 36 air communication port, 50
nucleic acid extraction container, 52 nucleic acid extraction solution injection port, 54 first branch channel, 60 nucleic acid extraction container, 62 nucleic acid extraction solution outlet, 64 second branch channel, 80
nucleic acid extraction container, 82 filter part 82a biological material collection area, 82b sample permeation area, 84 filter, 90 second channel, 100
nucleic acid extraction container, 102 nucleic acid extraction solution outlet, 104 second branch channel, 106
air injection port, 108 third branch channel, 120
nucleic acid extraction container, 124 first channel, 126 second channel, 128 third channel, 130 filter part, 130a biological material collection area, 130b
sample permeation area, 132 filter, 140 sample injection port, 146 sample discharge port, 148 air communication port, 160 nucleic acid extraction container, 164 channel, 166 filter part, 166a biological material collection area, 166b sample permeation area, 168 filter, 174 sample injection port, 176 sample discharge port, 178
air communication port

### [Sequence Listing Free Text]

Sequence number 1: Oncorhynchus mykiss forward PCR primer
Sequence number 2: Oncorhynchus mykiss reverse PCR primer
Sequence number 3: Oncorhynchus mykiss probe
Sequence number 4: Cyprinus carpio forward PCR primer
Sequence number 5: Cyprinus carpio reverse PCR primer
Sequence number 6: Cyprinus carpio probe
Sequence number 7: Katsuwonus pelamis forward PCR primer
Sequence number 8: Katsuwonus pelamis reverse PCR primer
Sequence number 9: Katsuwonus pelamis probe

### [Sequence Listing]

hairetsu.TXT

## Claims

1. A nucleic acid extraction container comprising:
a filter part that includes a hydrophilic filter for collecting biological materials containing a nucleic acid from a sample, a biological material collection area for collecting the biological materials on the filter, and a sample permeation area for allowing the passage of the sample having permeated through the filter;
a sample injection port that communicates with the biological material collection area;
an air communication port that communicates with the biological material collection area; and
a sample discharge port that communications with the sample permeation area, wherein
the air communication port is configured so as to be outwardly openable and closable.

2. The nucleic acid extraction container according to claim 1 wherein the biological material collection area has a channel shape.

3. The nucleic acid extraction container according to claim 1 or 2, wherein the sample injection port and the air communication port are arranged on opposite sides of each other across the biological material collection area.

4. The nucleic acid extraction container according to claim 1 or 2, further comprising:
a first channel that communicates the sample injection port with the biological material collection area;
a second channel that communicates the sample discharge port with the sample permeation area; and
a third channel that communicates the air communication port with the biological material collection area.

5. The nucleic acid extraction container according to claim 4, wherein the first channel and the third channel extend on opposite sides of each other across the biological material collection area.

6. The nucleic acid extraction container according to any one of claims 1 through 5, wherein the air communication port serves as a nucleic acid extraction solution injection port.

7. The nucleic acid extraction container according to claim 4 or 5, further comprising: a nucleic acid extraction solution injection port; and a first branch channel that communicates with the nucleic acid extraction solution injection port, wherein the first branch channel is connected to the third channel.

8. The nucleic acid extraction container according to any one of claims 4 through 7, further comprising: a nucleic acid extraction solution outlet; and a second branch channel that communicates with the nucleic acid extraction solution outlet, wherein the second branch channel is connected to the third channel.

9. The nucleic acid extraction container according to claim 8, further comprising: an air injection port; and a third branch channel that communicates with the air injection port, wherein the third branch channel is connected to the third channel.

10. The nucleic acid extraction container according to claim 9, wherein a region of the third channel between the connection position of the second branch channel and the third channel and the connection position of the third branch channel and the third channel is formed so as to dispense a predetermined amount of an extraction solution.

11. The nucleic acid extraction container according to any one of claims 4 through 10, wherein a nucleic acid extraction solution is enclosed in the third channel, and air is enclosed between a region where the nucleic acid extraction solution is present and the filter part.

12. The nucleic acid extraction container according to any one of claims 7 through 10, wherein a nucleic acid extraction solution is enclosed in the first branch channel, and air is enclosed between a region where the nucleic acid extraction solution is present and the filter part.

13. The nucleic acid extraction container according to claim 9 or 10, wherein a nucleic acid extraction solution is enclosed in the third branch channel, and air is enclosed between a region where the nucleic acid extraction solution is present and the filter part.

14. The nucleic acid extraction container according to any one of claims 4 through 13, wherein the average cross-sectional area of the third channel is smaller than the average cross-sectional area of the first channel.

15. The nucleic acid extraction container according to any one of claims 1 through 14, wherein the filter part is formed such that the presence or absence of a sample on the filter is visible from the outside.

16. A nucleic acid extraction method comprising:
passing a sample through a filter part provided with a hydrophilic filter and collecting biological materials containing a nucleic acid on the filter;
putting a nucleic acid extraction solution in the filter part and extracting the nucleic acid on the filter; and
collecting a solution containing the nucleic acid extracted on the filter.

17. The nucleic acid extraction method according to claim 16, wherein the extraction of the nucleic acid on the filter is performed by bringing the nucleic acid extraction solution into contact with a portion of the filter and moving the nucleic acid extraction solution such that the entire filter comes into contact with the nucleic acid extraction solution.

18. A nucleic acid extraction method comprising:
passing a sample through a filter part provided with a hydrophilic filter and collecting biological materials containing a nucleic acid on the filter;
putting a nucleic acid extraction solution in the filter part and extracting the nucleic acid on the filter;
moving a solution containing the nucleic acid extracted on the filter into a channel communicating with the filter part; and
dispensing the solution containing the nucleic acid by injecting air in the channel.

19. The nucleic acid extraction method according to claim 18, wherein the extraction of the nucleic acid on the filter is performed by bringing the nucleic acid extraction solution into contact with a portion of the filter and moving the nucleic acid extraction solution such that the entire filter comes into contact with the nucleic acid extraction solution.
